# EUROPEAN PATENT SPECIFICATION

(11) **EP 2 012 822 B1**
(45) Date of publication and mention of the grant of the patent: **20.01.2010**
(21) Application number: 07835738.1
(22) Date of filing: 27.04.2007
(51) Int. Cl.: A61K 39/12

(54) **MODIFIED ADENOVIRUS HEXON PROTEIN AND USES THEREOF**
MODIFIZIERTES ADENOVIRUS-HEXON-PROTEIN UND ANWENDUNGEN DAVON
PROTÉINE HEXON D'ADÉNOVIRUS MODIFIÉE ET SES UTILISATIONS

(30) Priority: 28.04.2006 US 796078 P
(43) Date of publication of application: 14.01.2009
(73) Proprietor: The Trustees of the University of Pennsylvania, Philadelphia, PA 19104 (US)
(72) Inventor: ROY, Soumitra, Wayne, PA 19087 (US); WILSON, James, M., Gladwyne, PA 19035 (US)
(74) Representative: Manaton, Ross Timothy
(86) International application number: PCT/US2007/010347
(87) International publication number: WO 2008/010864

(56) References cited:
- WO-A-2005/026337
- VIGNE E ET AL: "RGD inclusion in the hexon monomer provides adenovirus type-5 based vectors with a fiber knob-independent pathway for infection" JOURNAL OF VIROLOGY, vol. 73, no. 6, June 1999 (1999-06), pages 5156-5161, XP002126062 ISSN: 0022-538X cited in the application
- RUX J J ET AL: "Structural and phylogenetic analysis of adenovirus hexons by use of high-resolution x-ray crystallographic, molecular modeling, and sequence-based methods" JOURNAL OF VIROLOGY, THE AMERICAN SOCIETY FOR MICROBIOLOGY, US, vol. 77, no. 17, September 2003 (2003-09), pages 9553-9566, XP002385297 ISSN: 0022-538X cited in the application
- CRAWFORD-MIKSZA L ET AL: "ANALYSIS OF 15 ADENOVIRUS HEXON PROTEINS REVEALS THE LOCATION AND STRUCTURE OF SEVEN HYPERVARIABLE REGIONS CONTAINING SEROTYPE- SPECIFIC RESIDUES" JOURNAL OF VIROLOGY, vol. 70, no. 3, March 1996 (1996-03), pages 1836-1844, XP002071016 ISSN: 0022-538X cited in the application
- WORGALL STEFAN ET AL: "Protection against P. aeruginosa with an adenovirus vector containing an OprF epitope in the capsid" JOURNAL OF CLINICAL INVESTIGATION, vol. 115, no. 5, May 2005 (2005-05), pages 1281-1289, XP002469021 ISSN: 0021-9738

### SEQUENCE LISTING

<110> The Trustees of the University of Pennsylvania
   Roy, Soumitra
   Wilson, James M.
<120> MODIFIED ADENOVIRUS HEXON PROTEIN AND USES THEREOF
<130> UPN-R3807PCT
<150> US 60/796,078
   <151> 2006-04-28
<160> 28
<170> PatentIn version 3.4
<210> 1
   <211> 968
   <212> PRT
   <213> Human adenovirus type 2
<400> 1
   Met Ala Thr Pro Ser Met Met Pro Gln Trp Ser Tyr Met His Ile Ser
   1               5                   10                  15
   Gly Gln Asp Ala Ser Glu Tyr Leu Ser Pro Gly Leu Val Gln Phe Ala
               20                  25                  30
   Arg Ala Thr Glu Thr Tyr Phe Ser Leu Asn Asn Lys Phe Arg Asn Pro
           35                  40                  45
   Thr Val Ala Pro Thr His Asp Val Thr Thr Asp Arg Ser Gln Arg Leu
       50                  55                  60
   Thr Leu Arg Phe Ile Pro Val Asp Arg Glu Asp Thr Ala Tyr Ser Tyr
   65                  70                  75                  80
   Lys Ala Arg Phe Thr Leu Ala Val Gly Asp Asn Arg Val Leu Asp Met
                   85                  90                  95
   Ala Ser Thr Tyr Phe Asp Ile Arg Gly Val Leu Asp Arg Gly Pro Thr
               100                 105                 110
   Phe Lys Pro Tyr Ser Gly Thr Ala Tyr Asn Ala Leu Ala Pro Lys Gly
           115                 120                 125
   Ala Pro Asn Ser Cys Glu Trp Glu Gln Thr Glu Asp Ser Gly Arg Ala
       130                 135                 140
   Val Ala Glu Asp Glu Glu Glu Glu Asp Glu Asp Glu Glu Glu Glu Glu
   145                 150                 155                 160
   Glu Glu Gln Asn Ala Arg Asp Gln Ala Thr Lys Lys Thr His Val Tyr
                   165                 170                 175
   Ala Gln Ala Pro Leu Ser Gly Glu Thr Ile Thr Lys Ser Gly Leu Gln
               180                 185                 190
   Ile Gly Ser Asp Asn Ala Glu Thr Gln Ala Lys Pro Val Tyr Ala Asp
           195                 200                 205
   Pro Ser Tyr Gln Pro Glu Pro Gln Ile Gly Glu Ser Gln Trp Asn Glu
       210                 215                 220
   Ala Asp Ala Asn Ala Ala Gly Gly Arg Val Leu Lys Lys Thr Thr Pro
   225                 230                 235                 240
   Met Lys Pro Cys Tyr Gly Ser Tyr Ala Arg Pro Thr Asn Pro Phe Gly
                   245                 250                 255
   Gly Gln Ser Val Leu Val Pro Asp Glu Lys Gly Val Pro Leu Pro Lys
               260                 265                 270
   Val Asp Leu Gln Phe Phe Ser Asn Thr Thr Ser Leu Asn Asp Arg Gln
           275                 280                 285
   Gly Asn Ala Thr Lys Pro Lys Val Val Leu Tyr Ser Glu Asp Val Asn
       290                 295                 300
   Met Glu Thr Pro Asp Thr His Leu Ser Tyr Lys Pro Gly Lys Gly Asp
   305                 310                 315                 320
   Glu Asn Ser Lys Ala Met Leu Gly Gln Gln Ser Met Pro Asn Arg Pro
                   325                 330                 335
   Asn Tyr Ile Ala Phe Arg Asp Asn Phe Ile Gly Leu Met Tyr Tyr Asn
               340                 345                 350
   Ser Thr Gly Asn Met Gly Val Leu Ala Gly Gln Ala Ser Gln Leu Asn
   355 360 365
   Ala Val Val Asp Leu Gln Asp Arg Asn Thr Glu Leu Ser Tyr Gln Leu
   370 375 380
   Leu Leu Asp Ser Ile Gly Asp Arg Thr Arg Tyr Phe Ser Met Trp Asn
   385 390 395 400
   Gln Ala Val Asp Ser Tyr Asp Pro Asp Val Arg Ile Ile Glu Asn His
   405 410 415
   Gly Thr Glu Asp Glu Leu Pro Asn Tyr Cys Phe Pro Leu Gly Gly Ile
   420 425 430
   Gly Val Thr Asp Thr Tyr Gln Ala Ile Lys Ala Asn Gly Asn Gly Ser
   435 440 445
   Gly Asp Asn Gly Asp Thr Thr Trp Thr Lys Asp Glu Thr Phe Ala Thr
   450 455 460
   Arg Asn Glu Ile Gly Val Gly Asn Asn Phe Ala Met Glu Ile Asn Leu
   465 470 475 480
   Asn Ala Asn Leu Trp Arg Asn Phe Leu Tyr Ser Asn Ile Ala Leu Tyr
   485 490 495
   Leu Pro Asp Lys Leu Lys Tyr Asn Pro Thr Asn Val Glu Ile Ser Asp
   500 505 510
   Asn Pro Asn Thr Tyr Asp Tyr Met Asn Lys Arg Val Val Ala Pro Gly
   515 520 525
   Leu Val Asp Cys Tyr Ile Asn Leu Gly Ala Arg Trp Ser Leu Asp Tyr
   530 535 540
   Met Asp Asn Val Asn Pro Phe Asn His His Arg Asn Ala Gly Leu Arg
   545 550 555 560
   Tyr Arg Ser Met Leu Leu Gly Asn Gly Arg Tyr Val Pro Phe His Ile
   565 570 575
   Gln Val Pro Gln Lys Phe Phe Ala Ile Lys Asn Leu Leu Leu Leu Pro
   580 585 590
   Gly Ser Tyr Thr Tyr Glu Trp Asn Phe Arg Lys Asp Val Asn Met Val
   595 600 605
   Leu Gln Ser Ser Leu Gly Asn Asp Leu Arg Val Asp Gly Ala Ser Ile
   610 615 620
   Lys Phe Asp Ser Ile Cys Leu Tyr Ala Thr Phe Phe Pro Met Ala His
   625 630 635 640
   Asn Thr Ala Ser Thr Leu Glu Ala Met Leu Arg Asn Asp Thr Asn Asp
   645 650 655
   Gln Ser Phe Asn Asp Tyr Leu Ser Ala Ala Asn Met Leu Tyr Pro Ile
   660 665 670
   Pro Ala Asn Ala Thr Asn Val Pro Ile Ser Ile Pro Ser Arg Asn Trp
   675 680 685
   Ala Ala Phe Arg Gly Trp Ala Phe Thr Arg Leu Lys Thr Lys Glu Thr
   690 695 700
   Pro Ser Leu Gly Ser Gly Tyr Asp Pro Tyr Tyr Thr Tyr Ser Gly Ser
   705 710 715 720
   Ile Pro Tyr Leu Asp Gly Thr Phe Tyr Leu Asn His Thr Phe Lys Lys
   725 730 735
   Val Ala Ile Thr Phe Asp Ser Ser Val Ser Trp Pro Gly Asn Asp Arg
   740 745 750
   Leu Leu Thr Pro Asn Glu Phe Glu Ile Lys Arg Ser Val Asp Gly Glu
   755 760 765
   Gly Tyr Asn Val Ala Gln Cys Asn Met Thr Lys Asp Trp Phe Leu Val
   770 775 780
   Gln Met Leu Ala Asn Tyr Asn Ile Gly Tyr Gln Gly Phe Tyr Ile Pro
   785 790 795 800
   Glu Ser Tyr Lys Asp Arg Met Tyr Ser Phe Phe Arg Asn Phe Gln Pro
   805 810 815
   Met Ser Arg Gln Val Val Asp Asp Thr Lys Tyr Lys Glu Tyr Gln Gln
   820 825 830
   Val Gly Ile Leu His Gln His Asn Asn Ser Gly Phe Val Gly Tyr Leu
   835 840 845
   Ala Pro Thr Met Arg Glu Gly Gln Ala Tyr Pro Ala Asn Val Pro Tyr
   850 855 860
   Pro Leu Ile Gly Lys Thr Ala Val Asp Ser Ile Thr Gln Lys Lys Phe
   865 870 875 880
   Leu Cys Asp Arg Thr Leu Trp Arg Ile Pro Phe Ser Ser Asn Phe Met
   885 890 895
   Ser Met Gly Ala Leu Thr Asp Leu Gly Gln Asn Leu Leu Tyr Ala Asn
   900 905 910
   Ser Ala His Ala Leu Asp Met Thr Phe Glu Val Asp Pro Met Asp Glu
   915 920 925
   Pro Thr Leu Leu Tyr Val Leu Phe Glu Val Phe Asp Val Val Arg Val
   930 935 940
   His Gln Pro His Arg Gly Val Ile Glu Thr Val Tyr Leu Arg Thr Pro
   945 950 955 960
   Phe Ser Ala Gly Asn Ala Thr Thr
   965
<210> 2
   <211> 952
   <212> PRT
   <213> Human adenovirus type 5
<400> 2
   Met Ala Thr Pro Ser Met Met Pro Gln Trp Ser Tyr Met His Ile Ser
   1               5                   10                  15
   Gly Gln Asp Ala Ser Glu Tyr Leu Ser Pro Gly Leu Val Gln Phe Ala
               20                  25                  30
   Arg Ala Thr Glu Thr Tyr Phe Ser Leu Asn Asn Lys Phe Arg Asn Pro
           35                  40                  45
   Thr Val Ala Pro Thr His Asp Val Thr Thr Asp Arg Ser Gln Arg Leu
       50                  55                  60
   Thr Leu Arg Phe Ile Pro Val Asp Arg Glu Asp Thr Ala Tyr Ser Tyr
   65                  70                  75                  80
   Lys Ala Arg Phe Thr Leu Ala Val Gly Asp Asn Arg Val Leu Asp Met
                   85                  90                  95
   Ala Ser Thr Tyr Phe Asp Ile Arg Gly Val Leu Asp Arg Gly Pro Thr
               100                 105                 110
   Phe Lys Pro Tyr Ser Gly Thr Ala Tyr Asn Ala Leu Ala Pro Lys Gly
           115                 120                 125
   Ala Pro Asn Pro Cys Glu Trp Asp Glu Ala Ala Thr Ala Leu Glu Ile
       130                 135                 140
   Asn Leu Glu Glu Glu Asp Asp Asp Asn Glu Asp Glu Val Asp Glu Gln
   145                 150                 155                 160
   Ala Glu Gln Gln Lys Thr His Val Phe Gly Gln Ala Pro Tyr Ser Gly
                   165                 170                 175
   Ile Asn Ile Thr Lys Glu Gly Ile Gln Ile Gly Val Glu Gly Gln Thr
               180                 185                 190
   Pro Lys Tyr Ala Asp Lys Thr Phe Gln Pro Glu Pro Gln Ile Gly Glu
           195                 200                 205
   Ser Gln Trp Tyr Glu Thr Glu Ile Asn His Ala Ala Gly Arg Val Leu
       210                 215                 220
   Lys Lys Thr Thr Pro Met Lys Pro Cys Tyr Gly Ser Tyr Ala Lys Pro
   225                 230                 235                 240
   Thr Asn Glu Asn Gly Gly Gln Gly Ile Leu Val Lys Gln Gln Asn Gly
                   245                 250                 255
   Lys Leu Glu Ser Gln Val Glu Met Gln Phe Phe Ser Thr Thr Glu Ala
               260                 265                 270
   Thr Ala Gly Asn Gly Asp Asn Leu Thr Pro Lys Val Val Leu Tyr Ser
           275                 280                 285
   Glu Asp Val Asp Ile Glu Thr Pro Asp Thr His Ile Ser Tyr Met Pro
       290                 295                 300
   Thr Ile Lys Glu Gly Asn Ser Arg Glu Leu Met Gly Gln Gln Ser Met
   305                 310                 315                 320
   Pro Asn Arg Pro Asn Tyr Ile Ala Phe Arg Asp Asn Phe Ile Gly Leu
                   325                 330                 335
   Met Tyr Tyr Asn Ser Thr Gly Asn Met Gly Val Leu Ala Gly Gln Ala
               340                 345                 350
   Ser Gln Leu Asn Ala Val Val Asp Leu Gln Asp Arg Asn Thr Glu Leu
           355                 360                 365
   Ser Tyr Gln Leu Leu Leu Asp Ser Ile Gly Asp Arg Thr Arg Tyr Phe
       370                 375                 380
   Ser Met Trp Asn Gln Ala Val Asp Ser Tyr Asp Pro Asp Val Arg Ile
   385                 390                 395                 400
   Ile Glu Asn His Gly Thr Glu Asp Glu Leu Pro Asn Tyr Cys Phe Pro
                   405                 410                 415
   Leu Gly Gly Val Ile Asn Thr Glu Thr Leu Thr Lys Val Lys Pro Lys
               420                 425                 430
   Thr Gly Gln Glu Asn Gly Trp Glu Lys Asp Ala Thr Glu Phe Ser Asp
           435                 440                 445
   Lys Asn Glu Ile Arg Val Gly Asn Asn Phe Ala Met Glu Ile Asn Leu
   450 455 460
   Asn Ala Asn Leu Trp Arg Asn Phe Leu Tyr Ser Asn Ile Ala Leu Tyr
   465 470 475 480
   Leu Pro Asp Lys Leu Lys Tyr Ser Pro Ser Asn Val Lys Ile Ser Asp
   485 490 495
   Asn Pro Asn Thr Tyr Asp Tyr Met Asn Lys Arg Val Val Ala Pro Gly
   500 505 510
   Leu Val Asp Cys Tyr Ile Asn Leu Gly Ala Arg Trp Ser Leu Asp Tyr
   515 520 525
   Met Asp Asn Val Asn Pro Phe Asn His His Arg Asn Ala Gly Leu Arg
   530 535 540
   Tyr Arg Ser Met Leu Leu Gly Asn Gly Arg Tyr Val Pro Phe His Ile
   545 550 555 560
   Gln Val Pro Gln Lys Phe Phe Ala Ile Lys Asn Leu Leu Leu Leu Pro
   565 570 575
   Gly Ser Tyr Thr Tyr Glu Trp Asn Phe Arg Lys Asp Val Asn Met Val
   580 585 590
   Leu Gln Ser Ser Leu Gly Asn Asp Leu Arg Val Asp Gly Ala Ser Ile
   595 600 605
   Lys Phe Asp Ser Ile Cys Leu Tyr Ala Thr Phe Phe Pro Met Ala His
   610 615 620
   Asn Thr Ala Ser Thr Leu Glu Ala Met Leu Arg Asn Asp Thr Asn Asp
   625 630 635 640
   Gln Ser Phe Asn Asp Tyr Leu Ser Ala Ala Asn Met Leu Tyr Pro Ile
   645 650 655
   Pro Ala Asn Ala Thr Asn Val Pro Ile Ser Ile Pro Ser Arg Asn Trp
   660 665 670
   Ala Ala Phe Arg Gly Trp Ala Phe Thr Arg Leu Lys Thr Lys Glu Thr
   675 680 685
   Pro Ser Leu Gly Ser Gly Tyr Asp Pro Tyr Tyr Thr Tyr Ser Gly Ser
   690 695 700
   Ile Pro Tyr Leu Asp Gly Thr Phe Tyr Leu Asn His Thr Phe Lys Lys
   705 710 715 720
   Val Ala Ile Thr Phe Asp Ser Ser Val Ser Trp Pro Gly Asn Asp Arg
   725 730 735
   Leu Leu Thr Pro Asn Glu Phe Glu Ile Lys Arg Ser Val Asp Gly Glu
   740 745 750
   Gly Tyr Asn Val Ala Gln Cys Asn Met Thr Lys Asp Trp Phe Leu Val
   755 760 765
   Gln Met Leu Ala Asn Tyr Asn Ile Gly Tyr Gln Gly Phe Tyr Ile Pro
   770 775 780
   Glu Ser Tyr Lys Asp Arg Met Tyr Ser Phe Phe Arg Asn Phe Gln Pro
   785 790 795 800
   Met Ser Arg Gln Val Val Asp Asp Thr Lys Tyr Lys Asp Tyr Gln Gln
   805 810 815
   Val Gly Ile Leu His Gln His Asn Asn Ser Gly Phe Val Gly Tyr Leu
   820 825 830
   Ala Pro Thr Met Arg Glu Gly Gln Ala Tyr Pro Ala Asn Phe Pro Tyr
   835 840 845
   Pro Leu Ile Gly Lys Thr Ala Val Asp Ser Ile Thr Gln Lys Lys Phe
   850 855 860
   Leu Cys Asp Arg Thr Leu Trp Arg Ile Pro Phe Ser Ser Asn Phe Met
   865 870 875 880
   Ser Met Gly Ala Leu Thr Asp Leu Gly Gln Asn Leu Leu Tyr Ala Asn
   885 890 895
   Ser Ala His Ala Leu Asp Met Thr Phe Glu Val Asp Pro Met Asp Glu
   900 905 910
   Pro Thr Leu Leu Tyr Val Leu Phe Glu Val Phe Asp Val Val Arg Val
   915 920 925
   His Arg Pro His Arg Gly Val Ile Glu Thr Val Tyr Leu Arg Thr Pro
   930 935 940
   Phe Ser Ala Gly Asn Ala Thr Thr
   945 950
<210> 3
   <211> 942
   <212> PRT
   <213> Simian adenovirus
<400> 3
   Met Ala Thr Pro Ser Met Leu Pro Gln Trp Ala Tyr Met His Ile Ala
   1 5 10 15
   Gly Gln Asp Ala Ser Glu Tyr Leu Ser Pro Gly Leu Val Gln Phe Ala
   20 25 30
   Arg Ala Thr Asp Thr Tyr Phe Ser Leu Gly Asn Lys Phe Arg Asn Pro
   35 40 45
   Thr Val Ala Pro Thr His Asp Val Thr Thr Asp Arg Ser Gln Arg Leu
   50 55 60
   Thr Leu Arg Phe Val Pro Val Asp Arg Glu Asp Asn Thr Tyr Ser Tyr
   65 70 75 80
   Lys Val Arg Tyr Thr Leu Ala Val Gly Asp Asn Arg Val Leu Asp Met
   85 90 95
   Ala Ser Thr Tyr Phe Asp Ile Arg Gly Val Leu Asp Arg Gly Pro Ser
   100 105 110
   Phe Lys Pro Tyr Ser Gly Thr Ala Tyr Asn Ser Leu Ala Pro Lys Gly
   115 120 125
   Ala Pro Asn Ser Ser Gln Trp Glu Gln Ala Lys Thr Gly Asn Gly Gly
   130 135 140
   Thr Met Glu Thr His Thr Tyr Gly Val Ala Pro Met Gly Gly Glu Asn
   145 150 155 160
   Ile Thr Lys Asp Gly Leu Gln Ile Gly Thr Asp Val Thr Ala Asn Gln
   165 170 175
   Asn Lys Pro Ile Tyr Ala Asp Lys Thr Phe Gln Pro Glu Pro Gln Val
   180 185 190
   Gly Glu Glu Asn Trp Gln Glu Thr Glu Asn Phe Tyr Gly Gly Arg Ala
   195 200 205
   Leu Lys Lys Asp Thr Asn Met Lys Pro Cys Tyr Gly Ser Tyr Ala Arg
   210 215 220
   Pro Thr Asn Glu Lys Gly Gly Gln Ala Lys Leu Lys Val Gly Asp Asp
   225 230 235 240
   Gly Val Pro Thr Lys Glu Phe Asp Ile Asp Leu Ala Phe Phe Asp Thr
   245 250 255
   Pro Gly Gly Thr Val Asn Gly Gln Asp Glu Tyr Lys Ala Asp Ile Val
   260 265 270
   Met Tyr Thr Glu Asn Thr Tyr Leu Glu Thr Pro Asp Thr His Val Val
   275 280 285
   Tyr Lys Pro Gly Lys Asp Asp Ala Ser Ser Glu Ile Asn Leu Val Gln
   290 295 300
   Gln Ser Met Pro Asn Arg Pro Asn Tyr Ile Gly Phe Arg Asp Asn Phe
   305 310 315 320
   Ile Gly Leu Met Tyr Tyr Asn Ser Thr Gly Asn Met Gly Val Leu Ala
   325 330 335
   Gly Gln Ala Ser Gln Leu Asn Ala Val Val Asp Leu Gln Asp Arg Asn
   340 345 350
   Thr Glu Leu Ser Tyr Gln Leu Leu Leu Asp Ser Leu Gly Asp Arg Thr
   355 360 365
   Arg Tyr Phe Ser Met Trp Asn Gln Ala Val Asp Ser Tyr Asp Pro Asp
   370 375 380
   Val Arg Ile Ile Glu Asn His Gly Val Glu Asp Glu Leu Pro Asn Tyr
   385 390 395 400
   Cys Phe Pro Leu Asp Gly Ser Gly Thr Asn Ala Ala Tyr Gln Gly Val
   405 410 415
   Lys Val Lys Asp Gly Gln Asp Gly Asp Val Glu Ser Glu Trp Glu Asn
   420 425 430
   Asp Asp Thr Val Ala Ala Arg Asn Gln Leu Cys Lys Gly Asn Ile Phe
   435 440 445
   Ala Met Glu Ile Asn Leu Gln Ala Asn Leu Trp Arg Ser Phe Leu Tyr
   450 455 460
   Ser Asn Val Ala Leu Tyr Leu Pro Asp Ser Tyr Lys Tyr Thr Pro Thr
   465 470 475 480
   Asn Val Thr Leu Pro Thr Asn Thr Asn Thr Tyr Asp Tyr Met Asn Gly
   485 490 495
   Arg Val Thr Pro Pro Ser Leu Val Asp Ala Tyr Leu Asn Ile Gly Ala
   500 505 510
   Arg Trp Ser Leu Asp Pro Met Asp Asn Val Asn Pro Phe Asn His His
   515 520 525
   Arg Asn Ala Gly Leu Arg Tyr Arg Ser Met Leu Leu Gly Asn Gly Arg
   530 535 540
   Tyr Val Pro Phe His Ile Gln Val Pro Gln Lys Phe Phe Ala Ile Lys
   545 550 555 560
   Ser Leu Leu Leu Leu Pro Gly Ser Tyr Thr Tyr Glu Trp Asn Phe Arg
                   565                 570                 575
   Lys Asp Val Asn Met Ile Leu Gln Ser Ser Leu Gly Asn Asp Leu Arg
               580                 585                 590
   Thr Asp Gly Ala Ser Ile Ala Phe Thr Ser Ile Asn Leu Tyr Ala Thr
           595                 600                 605
   Phe Phe Pro Met Ala His Asn Thr Ala Ser Thr Leu Glu Ala Met Leu
       610                 615                 620
   Arg Asn Asp Thr Asn Asp Gln Ser Phe Asn Asp Tyr Leu Ser Ala Ala
   625                 630                 635                 640
   Asn Met Leu Tyr Pro Ile Pro Ala Asn Ala Thr Asn Val Pro Ile Ser
                   645                 650                 655
   Ile Pro Ser Arg Asn Trp Ala Ala Phe Arg Gly Trp Ser Phe Thr Arg
               660                 665                 670
   Leu Lys Thr Arg Glu Thr Pro Ser Leu Gly Ser Gly Phe Asp Pro Tyr
           675                 680                 685
   Phe Val Tyr Ser Gly Ser Ile Pro Tyr Leu Asp Gly Thr Phe Tyr Leu
       690                 695                 700
   Asn His Thr Phe Lys Lys Val Ser Ile Thr Phe Asp Ser Ser Val Ser
   705                 710                 715                 720
   Trp Pro Gly Asn Asp Arg Leu Leu Thr Pro Asn Glu Phe Glu Ile Lys
                   725                 730                 735
   Arg Thr Val Asp Gly Glu Gly Tyr Asn Val Ala Gln Cys Asn Met Thr
               740                 745                 750
   Lys Asp Trp Phe Leu Val Gln Met Leu Ala His Tyr Asn Ile Gly Tyr
           755                 760                 765
   Gln Gly Phe Tyr Val Pro Glu Gly Tyr Lys Asp Arg Met Tyr Ser Phe
   770 775 780
   Phe Arg Asn Phe Gln Pro Met Ser Arg Gln Val Val Asp Glu Val Asn
   785 790 795 800
   Tyr Lys Asp Tyr Gln Ala Val Thr Leu Ala Tyr Gln His Asn Asn Ser
   805 810 815
   Gly Phe Val Gly Tyr Leu Ala Pro Thr Met Arg Gln Gly Gln Pro Tyr
   820 825 830
   Pro Ala Asn Tyr Pro Tyr Pro Leu Ile Gly Lys Ser Ala Val Ala Ser
   835 840 845
   Val Thr Gln Lys Lys Phe Leu Cys Asp Arg Val Met Trp Arg Ile Pro
   850 855 860
   Phe Ser Ser Asn Phe Met Ser Met Gly Ala Leu Thr Asp Leu Gly Gln
   865 870 875 880
   Asp Met Leu Tyr Ala Asp Ser Ala His Ala Leu Asp Met Asn Phe Glu
   885 890 895
   Val Asp Pro Met Asp Glu Ser Thr Leu Leu Tyr Val Val Phe Glu Val
   900 905 910
   Phe Asp Val Val Arg Val His Gln Pro His Arg Gly Val Ile Glu Ala
   915 920 925
   Val Tyr Leu Arg Thr Pro Glu Ser Ala Gly Asn Ala Thr Thr
   930 935 940
<210> 4
   <211> 933
   <212> PRT
   <213> Simian adenovirus
<400> 4
   Met Ala Thr Pro Ser Met Leu Pro Gln Trp Ala Tyr Met His Ile Ala
   1 5 10 15
   Gly Gln Asp Ala Ser Glu Tyr Leu Ser Pro Gly Leu Val Gln Phe Ala
   20 25 30
   Arg Ala Thr Asp Thr Tyr Phe Ser Leu Gly Asn Lys Phe Arg Asn Pro
   35 40 45
   Thr Val Ala Pro Thr His Asp Val Thr Thr Asp Arg Ser Gln Arg Leu
   50 55 60
   Thr Leu Arg Phe Val Pro Val Asp Arg Glu Asp Asn Thr Tyr Ser Tyr
   65 70 75 80
   Lys Val Arg Tyr Thr Leu Ala Val Gly Asp Asn Arg Val Leu Asp Met
   85 90 95
   Ala Ser Thr Tyr Phe Asp Ile Arg Gly Val Leu Asp Arg Gly Pro Ser
   100 105 110
   Phe Lys Pro Tyr Ser Gly Thr Ala Tyr Asn Ser Leu Ala Pro Lys Gly
   115 120 125
   Ala Pro Asn Thr Cys Gln Trp Thr Tyr Lys Ala Asp Gly Asp Thr Gly
   130 135 140
   Thr Glu Lys Thr Tyr Thr Tyr Gly Asn Ala Pro Val Gln Gly Ile Ser
   145 150 155 160
   Ile Thr Lys Asp Gly Ile Gln Leu Gly Thr Asp Thr Asp Asp Gln Pro
   165 170 175
   Ile Tyr Ala Asp Lys Thr Tyr Gln Pro Glu Pro Gln Val Gly Asp Ala
   180 185 190
   Glu Trp His Asp Ile Thr Gly Thr Asp Glu Lys Tyr Gly Gly Arg Ala
   195 200 205
   Leu Lys Pro Asp Thr Lys Met Lys Pro Cys Tyr Gly Ser Phe Ala Lys
   210 215 220
   Pro Thr Asn Lys Glu Gly Gly Gln Ala Asn Val Lys Thr Glu Thr Gly
   225 230 235 240
   Gly Thr Lys Glu Tyr Asp Ile Asp Met Ala Phe Phe Asp Asn Arg Ser
                   245                 250                 255
   Ala Ala Ala Ala Gly Leu Ala Pro Glu Ile Val Leu Tyr Thr Glu Asn
               260                 265                 270
   Val Asp Leu Glu Thr Pro Asp Thr His Ile Val Tyr Lys Ala Gly Thr
           275                 280                 285
   Asp Asp Ser Ser Ser Ser Ile Asn Leu Gly Gln Gln Ser Met Pro Asn
       290                 295                 300
   Arg Pro Asn Tyr Ile Gly Phe Arg Asp Asn Phe Ile Gly Leu Met Tyr
   305                 310                 315                 320
   Tyr Asn Ser Thr Gly Asn Met Gly Val Leu Ala Gly Gln Ala Ser Gln
                   325                 330                 335
   Leu Asn Ala Val Val Asp Leu Gln Asp Arg Asn Thr Glu Leu Ser Tyr
               340                 345                 350
   Gln Leu Leu Leu Asp Ser Leu Gly Asp Arg Thr Arg Tyr Phe Ser Met
           355                 360                 365
   Trp Asn Gln Ala Val Asp Ser Tyr Asp Pro Asp Val Arg Ile Ile Glu
       370                 375                 380
   Asn His Gly Val Glu Asp Glu Leu Pro Asn Tyr Cys Phe Pro Leu Asp
   385                 390                 395                 400
   Ala Val Gly Arg Thr Asp Thr Tyr Gln Gly Ile Lys Ala Asn Gly Ala
                   405                 410                 415
   Asp Gln Thr Thr Trp Thr Lys Asp Asp Thr Val Asn Asp Ala Asn Glu
               420                 425                 430
   Leu Gly Lys Gly Asn Pro Phe Ala Met Glu Ile Asn Ile Gln Ala Asn
           435                 440                 445
   Leu Trp Arg Asn Phe Leu Tyr Ala Asn Val Ala Leu Tyr Leu Pro Asp
       450                 455                 460
   Ser Tyr Lys Tyr Thr Pro Ala Asn Ile Thr Leu Pro Thr Asn Thr Asn
   465 470 475 480
   Thr Tyr Asp Tyr Met Asn Gly Arg Val Val Ala Pro Ser Leu Val Asp
   485 490 495
   Ala Tyr Ile Asn Ile Gly Ala Arg Trp Ser Leu Asp Pro Met Asp Asn
   500 505 510
   Val Asn Pro Phe Asn His His Arg Asn Ala Gly Leu Arg Tyr Arg Ser
   515 520 525
   Met Leu Leu Gly Asn Gly Arg Tyr Val Pro Phe His Ile Gln Val Pro
   530 535 540
   Gln Lys Phe Phe Ala Ile Lys Ser Leu Leu Leu Leu Pro Gly Ser Tyr
   545 550 555 560
   Thr Tyr Glu Trp Asn Phe Arg Lys Asp Val Asn Met Ile Leu Gln Ser
   565 570 575
   Ser Leu Gly Asn Asp Leu Arg Thr Asp Gly Ala Ser Ile Ala Pro Thr
   580 585 590
   Ser Ile Asn Leu Tyr Ala Thr Phe Phe Pro Met Ala His Asn Thr Ala
   595 600 605
   Ser Thr Leu Glu Ala Met Leu Arg Asn Asp Thr Asn Asp Gln Ser Phe
   610 615 620
   Asn Asp Tyr Leu Ser Ala Ala Asn Met Leu Tyr Pro Ile Pro Ala Asn
   625 630 635 640
   Ala Thr Asn Val Pro Ile Ser Ile Pro Ser Arg Asn Trp Ala Ala Phe
   645 650 655
   Arg Gly Trp Ser Phe Thr Arg Leu Lys Thr Arg Glu Thr Pro Ser Leu
   660 665 670
   Gly Ser Gly Phe Asp Pro Tyr Phe Val Tyr Ser Gly Ser Ile Pro Tyr
   675 680 685
   Leu Asp Gly Thr Phe Tyr Leu Asn His Thr Phe Lys Lys Val Ser Ile
       690                 695                 700
   Thr Phe Asp Ser Ser Val Ser Trp Pro Gly Asn Asp Arg Leu Leu Thr
   705                 710                 715                 720
   Pro Asn Glu Phe Glu Ile Lys Arg Thr Val Asp Gly Glu Gly Tyr Asn
                   725                 730                 735
   Val Ala Gln Cys Asn Met Thr Lys Ala Trp Phe Leu Val Gln Met Leu
               740                 745                 750
   Ala His Tyr Asn Ile Gly Tyr Gln Gly Phe Tyr Val Pro Glu Gly Tyr
           755                 760                 765
   Leu Asp Arg Met Tyr Ser Phe Phe Arg Asn Phe Gln Pro Met Ser Arg
       770                 775                 780
   Gln Val Val Asp Glu Val Asn Tyr Lys Asp Tyr Gln Ala Val Thr Leu
   785                 790                 795                 800
   Ala Tyr Gln His Asn Asn Ser Gly Phe Val Gly Tyr Leu Ala Pro Thr
                   805                 810                 815
   Met Arg Gln Gly Gln Pro Tyr Pro Ala Asn Tyr Pro Tyr Pro Leu Ile
               820                 825                 830
   Gly Lys Ser Ala Val Ala Ser Val Thr Gln Lys Lys Phe Leu Cys Asp
           835                 840                 845
   Arg Val Met Trp Arg Ile Pro Phe Ser Ser Asn Phe Met Ser Met Gly
       850                 855                 860
   Ala Leu Thr Asp Leu Gly Gln Asn Met Leu Tyr Ala Asn Ser Ala His
   865                 870                 875                 880
   Ala Leu Asp Met Asn Phe Glu Val Asp Pro Met Asp Glu Ser Thr Leu
                   885                 890                 895
   Leu Tyr Val Val Phe Glu Val Phe Asp Val Val Arg Val His Gln Pro
   900 905 910
   His Arg Gly Val Ile Glu Ala Val Tyr Leu Arg Thr Pro Phe Ser Ala
   915 920 925
   Gly Asn Ala Thr Thr
   930
<210> 5
   <211> 932
   <212> PRT
   <213> Simian adenovirus
<400> 5
   Met Ala Thr Pro Ser Met Leu Pro Gln Trp Ala Tyr Met His Ile Ala
   1 5 10 15
   Gly Gln Asp Ala Ser Glu Tyr Leu Ser Pro Gly Leu Val Gln Phe Ala
   20 25 30
   Arg Ala Thr Asp Thr Tyr Phe Ser Leu Gly Asn Lys Phe Arg Asn Pro
   35 40 45
   Thr Val Ala Pro Thr His Asp Val Thr Thr Asp Arg Ser Gln Arg Leu
   50 55 60
   Thr Leu Arg Phe Val Pro Val Asp Arg Glu Asp Asn Thr Tyr Ser Tyr
   65 70 75 80
   Lys Val Arg Tyr Thr Leu Ala Val Gly Asp Asn Arg Val Leu Asp Met
   85 90 95
   Ala Ser Thr Tyr Phe Asp Ile Arg Gly Val Leu Asp Arg Gly Pro Ser
   100 105 110
   Phe Lys Pro Tyr Ser Gly Thr Ala Tyr Asn Ser Leu Ala Pro Lys Gly
   115 120 125
   Ala Pro Asn Thr Cys Gln Trp Thr Tyr Lys Ala Gly Asp Thr Asp Thr
   130 135 140
   Glu Lys Thr Tyr Thr Tyr Gly Asn Ala Pro Val Gln Gly Ile Ser Ile
   145                 150                 155                 160
   Thr Lys Asp Gly Ile Gln Leu Gly Thr Asp Ser Asp Gly Gln Ala Ile
                   165                 170                 175
   Tyr Ala Asp Glu Thr Tyr Gln Pro Glu Pro Gln Val Gly Asp Ala Glu
               180                 185                 190
   Trp His Asp Ile Thr Gly Thr Asp Glu Lys Tyr Gly Gly Arg Ala Leu
           195                 200                 205
   Lys Pro Asp Thr Lys Met Lys Pro Cys Tyr Gly Ser Phe Ala Lys Pro
       210                 215                 220
   Thr Asn Lys Glu Gly Gly Gln Ala Asn Val Lys Thr Glu Thr Gly Gly
   225                 230                 235                 240
   Thr Lys Glu Tyr Asp Ile Asp Met Ala Phe Phe Asp Asn Arg Ser Ala
                   245                 250                 255
   Ala Ala Ala Gly Leu Ala Pro Glu Ile Val Leu Tyr Thr Glu Asn Val
               260                 265                 270
   Asp Leu Glu Thr Pro Asp Thr His Ile Val Tyr Lys Ala Gly Thr Asp
           275                 280                 285
   Asp Ser Ser Ser Ser Ile Asn Leu Gly Gln Gln Ser Met Pro Asn Arg
       290                 295                 300
   Pro Asn Tyr Ile Gly Phe Arg Asp Asn Phe Ile Gly Leu Met Tyr Tyr
   305                 310                 315                 320
   Asn Ser Thr Gly Asn Met Gly Val Leu Ala Gly Gln Ala Ser Gln Leu
                   325                 330                 335
   Asn Ala Val Val Asp Leu Gln Asp Arg Asn Thr Glu Leu Ser Tyr Gln
               340                 345                 350
   Leu Leu Leu Asp Ser Leu Gly Asp Arg Thr Arg Tyr Phe Ser Met Trp
           355                 360                 365
   Asn Gln Ala Val Asp Ser Tyr Asp Pro Asp Val Arg Ile Ile Glu Asn
   370 375 380
   His Gly Val Glu Asp Glu Leu Pro Asn Tyr Cys Phe Pro Leu Asp Ala
   385 390 395 400
   Val Gly Arg Thr Asp Thr Tyr Gln Gly Ile Lys Ala Asn Gly Asp Asn
   405 410 415
   Gln Thr Thr Trp Thr Lys Asp Asp Thr Val Asn Asp Ala Asn Glu Leu
   420 425 430
   Gly Lys Gly Asn Pro Phe Ala Met Glu Ile Asn Ile Gln Ala Asn Leu
   435 440 445
   Trp Arg Asn Phe Leu Tyr Ala Asn Val Ala Leu Tyr Leu Pro Asp Ser
   450 455 460
   Tyr Lys Tyr Thr Pro Ala Asn Ile Thr Leu Pro Thr Asn Thr Asn Thr
   465 470 475 480
   Tyr Asp Tyr Met Asn Gly Arg Val Val Ala Pro Ser Leu Val Asp Ala
   485 490 495
   Tyr Ile Asn Ile Gly Ala Arg Trp Ser Leu Asp Pro Met Asp Asn Val
   500 505 510
   Asn Pro Phe Asn His His Arg Asn Ala Gly Leu Arg Tyr Arg Ser Met
   515 520 525
   Leu Leu Gly Asn Gly Arg Tyr Val Pro Phe His Ile Gln Val Pro Gln
   530 535 540
   Lys Phe Phe Ala Ile Lys Ser Leu Leu Leu Leu Pro Gly Ser Tyr Thr
   545 550 555 560
   Tyr Glu Trp Asn Phe Arg Lys Asp Val Asn Met Ile Leu Gln Ser Ser
   565 570 575
   Leu Gly Asn Asp Leu Arg Thr Asp Gly Ala Ser Ile Ala Phe Thr Ser
   580 585 590
   Ile Asn Leu Tyr Ala Thr Phe Phe Pro Met Ala His Asn Thr Ala Ser
   595 600 605
   Thr Leu Phe Ala Met Leu Arg Asn Asp Thr Asn Asp Gln Ser Phe Asn
   610 615 620
   Asp Tyr Leu Ser Ala Ala Asn Met Leu Tyr Pro Ile Pro Ala Asn Ala
   625 630 635 640
   Thr Asn Val Pro Ile Ser Ile Pro Ser Arg Asn Trp Ala Ala Phe Arg
   645 650 655
   Gly Trp Ser Phe Thr Arg Leu Lys Thr Arg Glu Thr Pro Ser Leu Gly
   660 665 670
   Ser Gly Phe Asp Pro Tyr Phe Val Tyr Ser Gly Ser Ile Pro Tyr Leu
   675 680 685
   Asp Gly Thr Phe Tyr Leu Asn His Thr Phe Lys Lys Val Ser Ile Thr
   690 695 700
   Phe Asp Ser Ser Val Ser Trp Pro Gly Asn Asp Arg Leu Leu Thr Pro
   705 710 715 720
   Asn Glu Phe Glu Ile Lys Arg Thr Val Asp Gly Glu Gly Tyr Asn Val
   725 730 735
   Ala Gln Cys Asn Met Thr Lys Asp Trp Phe Leu Val Gln Met Leu Ala
   740 745 750
   His Tyr Asn Ile Gly Tyr Gln Gly Phe Tyr Val Pro Glu Gly Tyr Lys
   755 760 765
   Asp Arg Met Tyr Ser Phe Phe Arg Asn Phe Gln Pro Met Ser Arg Gln
   770 775 780
   Val Val Asp Glu Val Asn Tyr Lys Asp Tyr Gln Ala Val Thr Leu Ala
   785 790 795 800
   Tyr Gln His Asn Asn Ser Gly Phe Val Gly Tyr Leu Ala Pro Thr Met
   805 810 815
   Arg Gln Gly Gln Pro Tyr Pro Ala Asn Tyr Pro Tyr Pro Leu Ile Gly
   820 825 830
   Lys Ser Ala Val Ala Ser Val Thr Gln Lys Lys Phe Leu Cys Asp Arg
   835 840 845
   Val Met Trp Arg Ile Pro Phe Ser Ser Asn Phe Met Ser Met Gly Ala
   850 855 860
   Leu Thr Asp Leu Gly Gln Asn Met Leu Tyr Ala Asn Ser Ala His Ala
   865 870 875 880
   Leu Asp Met Asn Phe Glu Val Asp Pro Met Asp Glu Ser Thr Leu Leu
   885 890 895
   Tyr Val Val Phe Glu Val Phe Asp Val Val Arg Val His Gln Pro His
   900 905 910
   Arg Gly Val Ile Glu Ala Val Tyr Leu Arg Thr Pro Phe Ser Ala Gly
   915 920 925
   Asn Ala Thr Thr
   930
<210> 6
   <211> 933
   <212> PRT
   <213> Simian adenovirus
<220>
   <221> MISC_FEATURE
   <222> (826) .. (826)
   <223> can be any amino acid
<220>
   <221> MISC_FEATURE
   <222> (922)..(922)
   <223> can be any amino acid
<400> 6
   Met Ala Thr Pro Ser Met Leu Pro Gln Trp Ala Tyr Met His Ile Ala
   1 5 10 15
   Gly Gln Asp Ala Ser Glu Tyr Leu Ser Pro Gly Leu Val Gln Phe Ala
   20 25 30
   Arg Ala Thr Asp Thr Tyr Phe Ser Leu Gly Asn Lys Phe Arg Asn Pro
   35 40 45
   Thr Val Ala Pro Thr His Asp Val Thr Thr Asp Arg Ser Gln Arg Leu
   50 55 60
   Thr Leu Arg Phe Val Pro Val Asp Arg Glu Asp Asn Thr Tyr Ser Tyr
   65 70 75 80
   Lys Val Arg Tyr Thr Leu Ala Val Gly Asp Asn Arg Val Leu Asp Met
   85 90 95
   Ala Ser Thr Tyr Phe Asp Ile Arg Gly Val Leu Asp Arg Gly Pro Ser
   100 105 110
   Phe Lys Pro Tyr Ser Gly Thr Ala Tyr Asn Ser Leu Ala Pro Lys Gly
   115 120 125
   Ala Pro Asn Thr Cys Gln Trp Thr Tyr Lys Ala Asp Gly Glu Thr Ala
   130 135 140
   Thr Glu Lys Thr Tyr Thr Tyr Gly Asn Ala Pro Val Gln Gly Ile Asn
   145 150 155 160
   Ile Thr Lys Asp Gly Ile Gln Leu Gly Thr Asp Thr Asp Asp Gln Pro
   165 170 175
   Ile Tyr Ala Asp Lys Thr Tyr Gln Pro Glu Pro Gln Val Gly Asp Ala
   180 185 190
   Glu Trp His Asp Ile Thr Gly Thr Asp Glu Lys Tyr Gly Gly Arg Ala
   195 200 205
   Leu Lys Pro Asp Thr Lys Met Lys Pro Cys Tyr Gly Ser Phe Ala Lys
   210 215 220
   Pro Thr Asn Lys Glu Gly Gly Gln Ala Asn Val Lys Thr Gly Thr Gly
   225 230 235 240
   Thr Thr Lys Glu Tyr Asp Ile Asp Met Ala Phe Phe Asp Asn Arg Ser
   245 250 255
   Ala Ala Ala Ala Gly Leu Ala Pro Glu Ile Val Leu Tyr Thr Glu Asn
   260 265 270
   Val Asp Leu Glu Thr Pro Asp Thr His Ile Val Tyr Lys Ala Gly Thr
   275 280 285
   Asp Asp Ser Ser Ser Ser Ile Asn Leu Gly Gln Gln Ala Met Pro Asn
   290 295 300
   Arg Pro Asn Tyr Ile Gly Phe Arg Asp Asn Phe Ile Gly Leu Met Tyr
   305 310 315 320
   Tyr Asn Ser Thr Gly Asn Met Gly Val Leu Ala Gly Gln Ala Ser Gln
   325 330 335
   Leu Asn Ala Val Val Asp Leu Gln Asp Arg Asn Thr Glu Leu Ser Tyr
   340 345 350
   Gln Leu Leu Leu Asp Ser Leu Gly Asp Arg Thr Arg Tyr Phe Ser Met
   355 360 365
   Trp Asn Gln Ala Val Asp Ser Tyr Asp Pro Asp Val Arg Ile Ile Glu
   370 375 380
   Asn His Gly Val Glu Asp Glu Leu Pro Asn Tyr Cys Phe Pro Leu Asp
   385 390 395 400
   Ala Val Gly Arg Thr Asp Thr Tyr Gln Gly Ile Lys Ala Asn Gly Thr
   405 410 415
   Asp Gln Thr Thr Trp Thr Lys Asp Asp Ser Val Asn Asp Ala Asn Glu
   420 425 430
   Ile Gly Lys Gly Asn Pro Phe Ala Met Glu Ile Asn Ile Gln Ala Asn
   435 440 445
   Leu Trp Arg Asn Phe Leu Tyr Ala Asn Val Ala Leu Tyr Leu Pro Asp
   450 455 460
   Ser Tyr Lys Tyr Thr Pro Ala Asn Val Thr Leu Pro Thr Asn Thr Asn
   465 470 475 480
   Thr Tyr Asp Tyr Met Asn Gly Arg Val Val Ala Pro Ser Leu Val Asp
   485 490 495
   Ser Tyr Ile Asn Ile Gly Ala Arg Trp Ser Leu Asp Pro Met Asp Asn
   500 505 510
   Val Asn Pro Phe Asn His His Arg Asn Ala Gly Leu Arg Tyr Arg Ser
   515 520 525
   Met Leu Leu Gly Asn Gly Arg Tyr Val Pro Phe His Ile Gln Val Pro
   530 535 540
   Gln Lys Phe Phe Ala Ile Lys Ser Leu Leu Leu Leu Pro Gly Ser Tyr
   545 550 555 560
   Thr Tyr Glu Trp Asn Phe Arg Lys Asp Val Asn Met Ile Leu Gln Ser
   565 570 575
   Ser Leu Gly Asn Asp Leu Arg Thr Asp Gly Ala Ser Ile Ser Phe Thr
   580 585 590
   Ser Ile Asn Leu Tyr Ala Thr Phe Phe Pro Met Ala His Asn Thr Ala
   595 600 605
   Ser Thr Leu Glu Ala Met Leu Arg Asn Asp Thr Asn Asp Gln Ser Phe
   610 615 620
   Asn Asp Tyr Leu Ser Ala Ala Asn Met Leu Tyr Pro Ile Pro Ala Asn
   625 630 635 640
   Ala Thr Asn Val Pro Ile Ser Ile Pro Ser Arg Asn Trp Ala Ala Phe
   645 650 655
   Arg Gly Trp Ser Phe Thr Arg Leu Lys Thr Lys Glu Thr Pro Ser Leu
   660 665 670
   Gly Ser Gly Phe Asp Pro Tyr Phe Val Tyr Ser Gly Ser Ile Pro Tyr
           675                 680                 685
   Leu Asp Gly Thr Phe Tyr Leu Asn His Thr Phe Lys Lys Val Ser Ile
       690                 695                 700
   Thr Phe Asp Ser Ser Val Ser Trp Pro Gly Asn Asp Arg Leu Leu Thr
   705                 710                 715                 720
   Pro Asn Glu Phe Glu Ile Lys Arg Thr Val Asp Gly Glu Gly Tyr Asn
                   725                 730                 735
   Val Ala Gln Cys Asn Met Thr Lys Asp Trp Phe Leu Val Gln Met Leu
               740                 745                 750
   Ala His Tyr Asn Ile Gly Tyr Gln Gly Phe Tyr Val Pro Glu Gly Tyr
           755                 760                 765
   Lys Asp Arg Met Tyr Ser Phe Phe Arg Asn Phe Gln Pro Met Ser Arg
       770                 775                 780
   Gln Val Val Asp Glu Val Asn Tyr Lys Asp Tyr Gln Ala Val Thr Leu
   785                 790                 795                 800
   Ala Tyr Gln His Asn Asn Ser Gly Phe Val Gly Tyr Leu Ala Pro Thr
                   805                 810                 815
   Met Arg Gln Gly Gln Pro Tyr Pro Ala Xaa Tyr Pro Tyr Pro Leu Ile
               820                 825                 830
   Gly Lys Ser Ala Val Thr Ser Val Thr Gln Lys Lys Phe Leu Cys Asp
           835                 840                 845
   Arg Val Met Trp Arg Ile Pro Phe Ser Ser Asn Phe Met Ser Met Gly
       850                 855                 860
   Ala Leu Thr Asp Leu Gly Gln Asn Met Leu Tyr Ala Asn Ser Ala His
   865                 870                 875                 880
   Ala Leu Asp Met Asn Phe Glu Val Asp Pro Met Asp Glu Ser Thr Leu
                   885                 890                 895
   Leu Tyr Val Val Phe Glu Val Phe Asp Val Val Arg Val His Gln Pro
   900 905 910
   His Arg Gly Val Ile Glu Ala Val Tyr Xaa Arg Thr Pro Phe Ser Ala
   915 920 925
   Gly Asn Ala Thr Thr
   930
<210> 7
   <211> 921
   <212> PRT
   <213> Simian adenovirus
<400> 7
   Met Ala Thr Pro Ser Met Met Pro Gln Trp Ser Tyr Met His Ile Ala
   1 5 10 15
   Gly Gln Asp Ala Ser Glu Tyr Leu Ser Pro Gly Leu Val Gln Phe Ala
   20 25 30
   Arg Ala Thr Asp Thr Tyr Phe Ser Leu Gly Asn Lys Phe Arg Asn Pro
   35 40 45
   Thr Val Ala Pro Thr His Asp Val Thr Thr Asp Arg Ser Gln Arg Leu
   50 55 60
   Thr Leu Arg Phe Val Pro Val Asp Arg Glu Asp Thr Ala Tyr Ser Tyr
   65 70 75 80
   Lys Val Arg Tyr Thr Leu Ala Val Gly Asp Asn Arg Val Leu Asp Met
   85 90 95
   Ala Ser Thr Tyr Phe Asp Ile Arg Gly Val Leu Asp Arg Gly Pro Ser
   100 105 110
   Phe Lys Pro Tyr Ser Gly Thr Ala Tyr Asn Ser Leu Ala Pro Lys Gly
   115 120 125
   Ala Pro Asn Pro Ser Glu Trp Thr Asp Thr Ser Asp Asn Lys Leu Lys
   130 135 140
   Ala Tyr Ala Gln Ala Pro Tyr Gln Ser Gln Gly Leu Thr Lys Asp Gly
   145 150 155 160
   Ile Gln Val Gly Leu Val Val Thr Glu Ser Gly Gln Thr Pro Gln Tyr
   165 170 175
   Ala Asn Lys Val Tyr Gln Pro Glu Pro Gln Ile Gly Glu Asn Gln Tyr
   180 185 190
   Asn Leu Glu Gln Glu Asp Lys Ala Ala Gly Arg Val Leu Lys Lys Asp
   195 200 205
   Thr Pro Met Phe Pro Cys Tyr Gly Ser Tyr Ala Arg Pro Thr Asn Glu
   210 215 220
   Gln Gly Gly Gln Ala Lys Asn Gln Glu Val Asp Leu Gln Phe Phe Ala
   225 230 235 240
   Thr Pro Gly Asp Thr Gln Asn Thr Ala Lys Val Val Leu Tyr Ala Glu
   245 250 255
   Asn Val Asn Leu Glu Thr Pro Asp Thr His Leu Val Phe Lys Pro Asp
   260 265 270
   Asp Asp Ser Thr Ser Ser Lys Leu Leu Leu Gly Gln Gln Ala Ala Pro
   275 280 285
   Asn Arg Pro Asn Tyr Ile Gly Phe Arg Asp Asn Phe Ile Gly Leu Met
   290 295 300
   Tyr Tyr Asn Ser Thr Gly Asn Met Gly Val Leu Ala Gly Gln Ala Ser
   305 310 315 320
   Gln Leu Asn Ala Val Val Asp Leu Gln Asp Arg Asn Thr Glu Leu Ser
   325 330 335
   Tyr Gln Leu Met Leu Asp Ala Leu Gly Asp Arg Ser Arg Tyr Phe Ser
   340 345 350
   Met Trp Asn Gln Ala Val Asp Ser Tyr Asp Pro Asp Val Arg Ile Ile
   355 360 365
   Glu Asn His Gly Val Glu Asp Glu Leu Pro Asn Tyr Cys Phe Pro Leu
   370 375 380
   Gly Gly Met Val Val Thr Asp Asn Tyr Asn Ser Val Thr Pro Gln Asn
   385 390 395 400
   Gly Gly Ser Gly Asn Thr Trp Gln Ala Asp Asn Thr Thr Phe Ser Gln
   405 410 415
   Arg Gly Ala Gln Ile Gly Ser Gly Asn Met Phe Ala Leu Glu Ile Asn
   420 425 430
   Leu Gln Ala Asn Leu Trp Arg Gly Phe Leu Tyr Ser Asn Ile Gly Leu
   435 440 445
   Tyr Leu Pro Asp Ser Leu Lys Ile Thr Pro Asp Asn Ile Thr Leu Pro
   450 455 460
   Glu Asn Lys Asn Thr Tyr Gln Tyr Met Asn Gly Arg Val Thr Pro Pro
   465 470 475 480
   Gly Leu Ile Asp Thr Tyr Val Asn Val Gly Ala Arg Trp Ser Pro Asp
   485 490 495
   Val Met Asp Ser Ile Asn Pro Phe Asn His His Arg Asn Ala Gly Leu
   500 505 510
   Arg Tyr Arg Ser Met Leu Leu Gly Asn Gly Arg Tyr Val Pro Phe His
   515 520 525
   Ile Gln Val Pro Gln Lys Phe Phe Ala Ile Lys Asn Leu Leu Leu Leu
   530 535 540
   Pro Gly Ser Tyr Thr Tyr Glu Trp Asn Phe Arg Lys Asp Val Asn Met
   545 550 555 560
   Ile Leu Gln Ser Ser Leu Gly Asn Asp Leu Arg Val Asp Gly Ala Ser
   565 570 575
   Ile Arg Phe Asp Ser Ile Asn Leu Tyr Ala Asn Phe Phe Pro Met Ala
                   580                 585                 590
   His Asn Thr Ala Ser Thr Leu Glu Ala Met Leu Arg Asn Asp Thr Asn
           595                 600                 605
   Asp Gln Ser Phe Asn Asp Tyr Leu Cys Ala Ala Asn Met Leu Tyr Pro
       610                 615                 620
   Ile Pro Ala Asn Ala Thr Ser Val Pro Ile Ser Ile Pro Ser Arg Asn
   625                 630                 635                 640
   Trp Ala Ala Phe Arg Gly Trp Ser Phe Thr Arg Leu Lys Thr Lys Glu
                   645                 650                 655
   Thr Pro Ser Leu Gly Ser Gly Phe Asp Pro Tyr Phe Val Tyr Ser Gly
               660                 665                 670
   Ser Ile Pro Tyr Leu Asp Gly Thr Phe Tyr Leu Asn His Thr Phe Lys
           675                 680                 685
   Lys Val Ser Ile Met Phe Asp Ser Ser Val Ser Trp Pro Gly Asn Asp
       690                 695                 700
   Arg Leu Leu Thr Pro Asn Glu Phe Glu Ile Lys Arg Ser Val Asp Gly
   705                 710                 715                 720
   Glu Gly Tyr Asn Val Ala Gln Ser Asn Met Thr Lys Asp Trp Phe Leu
                   725                 730                 735
   Ile Gln Met Leu Ser His Tyr Asn Ile Gly Tyr Gln Gly Phe Tyr Val
               740                 745                 750
   Pro Glu Asn Tyr Lys Asp Arg Met Tyr Ser Phe Phe Arg Asn Phe Gln
           755                 760                 765
   Pro Met Ser Arg Gln Val Val Asp Thr Val Thr Tyr Thr Asp Tyr Lys
       770                 775                 780
   Asp Val Lys Leu Pro Tyr Gln His Asn Asn Ser Gly Phe Val Gly Tyr
   785                 790                 795                 800
   Met Gly Pro Thr Met Arg Glu Gly Gln Ala Tyr Pro Ala Asn Tyr Pro
   805 810 815
   Tyr Pro Leu Ile Gly Glu Thr Ala Val Pro Ser Leu Thr Gln Lys Lys
   820 825 830
   Phe Leu Cys Asp Arg Val Met Trp Arg Ile Pro Phe Ser Ser Asn Phe
   835 840 845
   Met Ser Met Gly Ser Leu Thr Asp Leu Gly Gln Asn Met Leu Tyr Ala
   850 855 860
   Asn Ser Ala His Ala Leu Asp Met Thr Phe Glu Val Asp Pro Met Asp
   865 870 875 880
   Glu Pro Thr Leu Leu Tyr Val Leu Phe Glu Val Phe Asp Val Val Arg
   885 890 895
   Ile His Gln Pro His Arg Gly Val Ile Glu Ala Val Tyr Leu Arg Thr
   900 905 910
   Pro Phe Ser Ala Gly Asn Ala Thr Thr
   915 920
<210> 8
   <211> 917
   <212> PRT
   <213> Simian adenovirus
<400> 8
   Met Ala Thr Pro Ser Met Met Pro Gln Trp Ser Tyr Met His Ile Ala
   1 5 10 15
   Gly Gln Asp Ala Ser Glu Tyr Leu Ser Pro Gly Leu Val Gln Phe Ala
   20 25 30
   Arg Ala Thr Glu Thr Tyr Phe Ser Leu Gly Asn Lys Phe Arg Asn Pro
   35 40 45
   Thr Val Ala Pro Thr His Asp Val Thr Thr Asp Arg Ser Gln Arg Leu
   50 55 60
   Thr Ile Arg Phe Val Pro Val Asp Lys Glu Asp Thr Ala Tyr Ser Tyr
   65                  70                  75                  80
   Lys Thr Arg Phe Thr Leu Ala Val Gly Asp Asn Arg Val Leu Asp Met
                   85                  90                  95
   Ala Ser Thr Tyr Phe Asp Ile Arg Gly Val Ile Asp Arg Gly Pro Ser
               100                 105                 110
   Phe Lys Pro Tyr Ser Gly Thr Ala Tyr Asn Ser Leu Ala Pro Lys Gly
           115                 120                 125
   Ala Pro Asn Asn Ser Gln Trp Asn Ala Thr Asp Asn Gly Asn Lys Pro
       130                 135                 140
   Val Cys Phe Ala Gln Ala Ala Phe Ile Gly Gln Ser Ile Thr Lys Asp
   145                 150                 155                 160
   Gly Val Gln Ile Gln Asn Ser Glu Asn Gln Gln Ala Ala Ala Asp Lys
                   165                 170                 175
   Thr Tyr Gln Pro Glu Pro Gln Ile Gly Val Ser Thr Trp Asp Thr Asn
               180                 185                 190
   Val Thr Ser Asn Ala Ala Gly Arg Val Leu Lys Ala Thr Thr Pro Met
           195                 200                 205
   Leu Pro Cys Tyr Gly Ser Tyr Ala Asn Pro Thr Asn Pro Asn Gly Gly
       210                 215                 220
   Gln Ala Lys Thr Glu Gly Asp Ile Ser Leu Asn Phe Phe Thr Thr Thr
   225                 230                 235                 240
   Ala Ala Ala Asp Asn Asn Pro Lys Val Val Leu Tyr Ser Glu Asp Val
                   245                 250                 255
   Asn Leu Gln Ala Pro Asp Thr His Leu Val Tyr Lys Pro Thr Val Gly
               260                 265                 270
   Glu Asn Val Ile Ala Ala Glu Ala Leu Leu Thr Gln Gln Ala Cys Pro
           275                 280                 285
   Asn Arg Ala Asn Tyr Ile Gly Phe Arg Asp Asn Phe Ile Gly Leu Met
       290                 295                 300
   Thr Thr Asn Ser Thr Gly Asn Met Gly Val Leu Ala Gly Gln Ala Ser
   305                 310                 315                 320
   Gln Leu Asn Ala Val Val Asp Leu Gln Asp Arg Asn Thr Glu Leu Ser
                   325                 330                 335
   Tyr Gln Leu Met Leu Asp Ala Leu Gly Asp Arg Thr Arg Tyr Phe Ser
               340                 345                 350
   Met Trp Asn Gln Ala Val Asp Ser Tyr Asp Pro Asp Val Arg Ile Ile
           355                 360                 365
   Glu Asn His Gly Val Glu Asp Glu Leu Pro Asn Tyr Cys Phe Pro Leu
       370                 375                 380
   Pro Gly Met Gly Ile Phe Asn Ser Tyr Lys Gly Val Lys Pro Gln Asn
   385                 390                 395                 400
   Gly Gly Asn Gly Asn Trp Glu Ala Asn Gly Asp Leu Ser Asn Ala Asn
                   405                 410                 415
   Glu Ile Ala Leu Gly Asn Ile Phe Ala Met Glu Ile Asn Leu His Ala
               420                 425                 430
   Asn Leu Trp Arg Ser Phe Leu Tyr Ser Asn Val Ala Leu Tyr Leu Pro
           435                 440                 445
   Asp Ser Tyr Lys Phe Thr Pro Ala Asn Ile Thr Leu Pro Ala Asn Gln
       450                 455                 460
   Asn Thr Tyr Gly Tyr Ile Asn Gly Arg Val Thr Ser Pro Thr Leu Val
   465                 470                 475                 480
   Asp Thr Phe Val Asn Ile Gly Ala Arg Trp Ser Pro Asp Pro Met Asp
                   485                 490                 495
   Asn Val Asn Pro Phe Asn His His Arg Asn Ala Gly Leu Arg Tyr Arg
               500                 505                 510
   Ser Met Leu Leu Gly Asn Gly Arg Val Val Pro Phe His Ile Gln Val
   515 520 525
   Pro Gln Lys Phe Phe Ala Ile Lys Asn Leu Leu Leu Leu Pro Gly Ser
   530 535 540
   Tyr Thr Tyr Glu Trp Ser Phe Arg Lys Asp Val Asn Met Ile Leu Gln
   545 550 555 560
   Ser Thr Leu Gly Asn Asp Leu Arg Val Asp Gly Ala Ser Val Arg Ile
   565 570 575
   Asp Ser Val Asn Leu Tyr Ala Asn Phe Phe Pro Met Ala His Asn Thr
   580 585 590
   Ala Ser Thr Leu Glu Ala Met Leu Arg Asn Asp Thr Asn Asp Gly Ser
   595 600 605
   Phe Asn Asp Tyr Leu Ser Ala Ala Asn Met Leu Tyr Pro Ile Pro Ala
   610 615 620
   Asn Ala Thr Asn Val Pro Ile Ser Ile Pro Ser Arg Asn Trp Ala Ala
   625 630 635 640
   Phe Arg Gly Trp Ser Phe Thr Arg Leu Lys Ala Lys Glu Thr Pro Ser
   645 650 655
   Leu Gly Ser Gly Phe Asp Pro Tyr Phe Val Tyr Ser Gly Thr Ile Pro
   660 665 670
   Tyr Leu Asp Gly Ser Phe Tyr Leu Asn His Thr Phe Lys Arg Leu Ser
   675 680 685
   Ile Met Phe Asp Ser Ser Val Ser Trp Pro Gly Asn Asp Arg Leu Leu
   690 695 700
   Thr Pro Asn Glu Phe Glu Ile Lys Arg Ile Val Asp Gly Glu Gly Tyr
   705 710 715 720
   Asn Val Ala Gln Ser Asn Met Thr Lys Asp Trp Phe Leu Ile Gln Met
   725 730 735
   Leu Ser His Tyr Asn Ile Gly Tyr Gln Gly Phe Tyr Val Pro Glu Gly
   740 745 750
   Tyr Lys Asp Arg Met Tyr Ser Phe Phe Arg Asn Phe Gln Pro Met Ser
   755 760 765
   Arg Gln Val Pro Asp Pro Thr Ala Ala Gly Tyr Gln Ala Val Pro Leu
   770 775 780
   Pro Arg Gln His Asn Asn Ser Gly Phe Val Gly Tyr Met Gly Pro Thr
   785 790 795 800
   Met Arg Glu Gly Gln Pro Tyr Pro Ala Asn Tyr Pro Tyr Pro Leu Ile
   805 810 815
   Gly Ala Thr Ala Val Pro Ala Ile Thr Gln Lys Lys Phe Leu Cys Asp
   820 825 830
   Arg Val Met Trp Arg Ile Pro Phe Ser Ser Asn Phe Met Ser Met Gly
   835 840 845
   Ala Leu Thr Asp Leu Gly Gln Asn Met Leu Tyr Ala Asn Ser Ala His
   850 855 860
   Ala Leu Asp Met Thr Phe Glu Val Asp Pro Met Asn Glu Pro Thr Leu
   865 870 875 880
   Leu Tyr Met Leu Phe Glu Val Phe Asp Val Val Arg Val His Gln Pro
   885 890 895
   His Arg Gly Ile Ile Glu Ala Val Tyr Leu Arg Thr Pro Phe Ser Ala
   900 905 910
   Gly Asn Ala Thr Thr
   915
<210> 9
   <211> 917
   <212> PRT
   <213> Simian adenovirus
<400> 9
   Met Ala Thr Pro Ser Met Met Pro Gln Trp Ser Tyr Met His Ile Ala
   1 5 10 15
   Gly Gln Asp Ala Ser Glu Tyr Leu Ser Pro Gly Leu Val Gln Phe Ala
   20 25 30
   Arg Ala Thr Asp Thr Tyr Phe Ser Leu Gly Asn Lys Phe Arg Asn Pro
   35 40 45
   Thr Val Ala Pro Thr His Asp Val Thr Thr Asp Arg Ser Gln Arg Leu
   50 55 60
   Thr Leu Arg Phe Val Pro Val Asp Arg Glu Asp Thr Ala Tyr Ser Tyr
   65 70 75 80
   Lys Val Arg Phe Thr Leu Ala Val Gly Asp Asn Arg Val Leu Asp Met
   85 90 95
   Ala Ser Thr Tyr Phe Asp Ile Arg Gly Val Leu Asp Arg Gly Pro Ser
   100 105 110
   Phe Lys Pro Tyr Ser Gly Thr Ala Tyr Asn Ser Leu Ala Pro Lys Gly
   115 120 125
   Ala Pro Asn Pro Ser Glu Trp Lys Gly Ser Asp Asn Lys Ile Ser Val
   130 135 140
   Arg Gly Gln Ala Pro Phe Phe Ser Thr Ser Ile Thr Lys Asp Gly Ile
   145 150 155 160
   Gln Val Ala Thr Asp Thr Ser Ser Gly Ala Val Tyr Ala Lys Lys Glu
   165 170 175
   Tyr Gln Pro Glu Pro Gln Val Gly Gln Glu Gln Trp Asn Ser Glu Ala
   180 185 190
   Ser Asp Ser Asp Lys Val Ala Gly Arg Ile Leu Lys Asp Thr Thr Pro
   195 200 205
   Met Phe Pro Cys Tyr Gly Ser Tyr Ala Lys Pro Thr Asn Glu Gln Gly
       210                 215                 220
   Gly Gln Gly Thr Asn Thr Val Asp Leu Gln Phe Phe Ala Ser Ser Ser
   225                 230                 235                 240
   Ala Thr Ser Thr Pro Lys Ala Val Leu Tyr Ala Glu Asp Val Ala Ile
                   245                 250                 255
   Glu Ala Pro Asp Thr His Leu Val Tyr Lys Pro Ala Val Thr Thr Thr
               260                 265                 270
   Thr Thr Ser Ser Gln Asp Leu Leu Thr Gln Gln Ala Ala Pro Asn Arg
           275                 280                 285
   Pro Asn Tyr Ile Gly Phe Arg Asp Asn Phe Ile Gly Leu Met Tyr Tyr
       290                 295                 300
   Asn Ser Thr Gly Asn Met Gly Val Leu Ala Gly Gln Ala Ser Gln Leu
   305                 310                 315                 320
   Asn Ala Val Val Asp Leu Gln Asp Arg Asn Thr Glu Leu Ser Tyr Gln
                   325                 330                 335
   Leu Met Leu Asp Ala Leu Gly Asp Arg Ser Arg Tyr Phe Ser Met Trp
               340                 345                 350
   Asn Gln Ala Val Asp Ser Tyr Asp Pro Asp Val Arg Ile Ile Glu Asn
           355                 360                 365
   His Gly Val Glu Asp Glu Leu Pro Asn Tyr Cys Phe Pro Leu Gly Gly
       370                 375                 380
   Ser Leu Val Thr Glu Thr Tyr Thr Gly Leu Ser Pro Gln Asn Gly Ser
   385                 390                 395                 400
   Asn Thr Trp Thr Thr Asp Ser Thr Thr Tyr Ala Thr Arg Gly Val Glu
                   405                 410                 415
   Ile Gly Ser Gly Asn Met Phe Ala Met Glu Ile Asn Leu Ala Ala Asn
               420                 425                 430
   Leu Trp Arg Ser Phe Leu Tyr Ser Asn Val Ala Leu Tyr Leu Pro Asp
           435                 440                 445
   Glu Tyr Lys Leu Thr Pro Asp Asn Ile Thr Leu Pro Asp Asn Lys Asn
       450                 455                 460
   Thr Tyr Asp Tyr Met Asp Gly Arg Val Ala Ala Pro Ser Ser Leu Asp
   465                 470                 475                 480
   Thr Tyr Val Asn Ile Gly Ala Arg Trp Ser Pro Asp Pro Met Asp Asn
                   485                 490                 495
   Val Asn Pro Phe Asn His His Arg Asn Ala Gly Leu Arg Tyr Arg Ser
               500                 505                 510
   Met Leu Leu Gly Asn Gly Arg Tyr Val Pro Phe His Ile Gln Val Pro
           515                 520                 525
   Gln Lys Phe Phe Ala Ile Lys Asn Leu Leu Leu Leu Pro Gly Ser Tyr
       530                 535                 540
   Thr Tyr Glu Trp Asn Phe Arg Lys Asp Val Asn Met Ile Leu Gln Ser
   545                 550                 555                 560
   Ser Leu Gly Asn Asp Leu Arg Val Asp Gly Ala Ser Val Arg Phe Asp
                   565                 570                 575
   Ser Ile Asn Leu Tyr Ala Asn Phe Phe Pro Met Ala His Asn Thr Ala
               580                 585                 590
   Ser Thr Leu Glu Ala Met Leu Arg Asn Asp Thr Asn Asp Gln Ser Phe
           595                 600                 605
   Asn Asp Tyr Leu Cys Ala Ala Asn Met Leu Tyr Pro Ile Pro Ala Asn
       610                 615                 620
   Ala Thr Ser Val Pro Ile Ser Ile Pro Ser Arg Asn Trp Ala Ala Phe
   625                 630                 635                 640
   Arg Gly Trp Ser Phe Thr Arg Leu Lys Thr Lys Glu Thr Pro Ser Leu
                   645                 650                 655
   Gly Ser Gly Phe Asp Pro Tyr Phe Thr Tyr Ser Gly Ser Ile Pro Tyr
   660 665 670
   Leu Asp Gly Thr Phe Tyr Leu Asn His Thr Phe Lys Lys Val Ser Ile
   675 680 685
   Met Phe Asp Ser Ser Val Ser Trp Pro Gly Asn Asp Arg Leu Leu Thr
   690 695 700
   Pro Asn Glu Phe Glu Ile Lys Arg Thr Val Asp Gly Glu Gly Tyr Asn
   705 710 715 720
   Val Ala Gln Cys Asn Met Thr Lys Asp Trp Phe Leu Ile Gln Met Leu
   725 730 735
   Ser His Tyr Asn Ile Gly Tyr Gln Gly Pro Tyr Val Pro Glu Gly Tyr
   740 745 750
   Lys Asp Arg Met Tyr Ser Phe Phe Arg Asn Phe Gln Pro Met Ser Arg
   755 760 765
   Gln Val Val Asp Thr Thr Thr Tyr Thr Asp Tyr Lys Asn Val Thr Leu
   770 775 780
   Pro Phe Gln His Asn Asn Ser Gly Phe Val Gly Tyr Met Gly Pro Thr
   785 790 795 800
   Met Arg Glu Gly Gln Ala Tyr Pro Ala Asn Tyr Pro Tyr Pro Leu Ile
   805 810 815
   Gly Lys Thr Ala Val Pro Ser Leu Thr Gln Lys Lys Phe Leu Cys Asp
   820 825 830
   Arg Thr Met Trp Arg Ile Pro Phe Ser Ser Asn Phe Met Ser Met Gly
   835 840 845
   Ala Leu Thr Asp Leu Gly Gln Asn Met Leu Tyr Ala Asn Ser Ala His
   850 855 860
   Ala Leu Asp Met Thr Phe Glu Val Asp Pro Met Asp Glu Pro Thr Leu
   865 870 875 880
   Leu Tyr Val Leu Phe Glu Val Phe Asp Val Val Arg Ile His Gln Pro
   885 890 895
   His Arg Gly Val Ile Glu Ala Val Tyr Leu Arg Thr Pro Phe Ser Ala
   900 905 910
   Gly Asn Ala Thr Thr
   915
<210> 10
   <211> 931
   <212> PRT
   <213> Simian adenovirus
<400> 10
   Met Ala Thr Pro Ser Met Met Pro Gln Trp Ser Tyr Met His Ile Ala
   1 5 10 15
   Gly Gln Asp Ala Ser Glu Tyr Leu Ser Pro Gly Leu Val Gln Phe Ala
   20 25 30
   Arg Ala Thr Asp Thr Tyr Phe Ser Leu Gly Asn Lys Phe Arg Asn Pro
   35 40 45
   Thr Val Ala Pro Thr His Asp Val Thr Thr Asp Arg Ser Gln Arg Leu
   50 55 60
   Thr Leu Arg Phe Val Pro Val Asp Arg Glu Asp Thr Ala Tyr Ser Tyr
   65 70 75 80
   Lys Val Arg Tyr Thr Leu Ala Val Gly Asp Asn Arg Val Leu Asp Met
   85 90 95
   Ala Ser Thr Tyr Phe Asp Ile Arg Gly Val Leu Asp Arg Gly Pro Ser
   100 105 110
   Phe Lys Pro Tyr Ser Gly Thr Ala Tyr Asn Ser Leu Ala Pro Lys Gly
   115 120 125
   Ala Pro Asn Pro Ala Glu Trp Thr Asn Ser Asp Ser Lys Val Lys Val
   130 135 140
   Arg Ala Gln Ala Pro Phe Val Ser Ser Tyr Gly Ala Thr Ala Ile Thr
   145 150 155 160
   Lys Glu Gly Ile Gln Val Gly Val Thr Leu Thr Asp Ser Gly Ser Thr
   165 170 175
   Pro Gln Tyr Ala Asp Lys Thr Tyr Gln Pro Glu Pro Gln Ile Gly Glu
   180 185 190
   Leu Gln Trp Asn Ser Asp Val Gly Thr Asp Asp Lys Ile Ala Gly Arg
   195 200 205
   Val Leu Lys Lys Thr Thr Pro Met Phe Pro Cys Tyr Gly Ser Tyr Ala
   210 215 220
   Arg Pro Thr Asn Glu Lys Gly Gly Gln Ala Thr Pro Ser Ala Ser Gln
   225 230 235 240
   Asp Val Gln Asn Pro Glu Leu Gln Phe Phe Ala Ser Thr Asn Val Ala
   245 250 255
   Asn Thr Pro Lys Ala Val Leu Thr Ala Glu Asp Val Ser Ile Glu Ala
   260 265 270
   Pro Asp Thr His Leu Val Phe Lys Pro Thr Val Thr Glu Gly Ile Thr
   275 280 285
   Ser Ser Glu Ala Leu Leu Thr Gln Gln Ala Ala Pro Asn Arg Pro Asn
   290 295 300
   Tyr Ile Ala Phe Arg Asp Asn Phe Ile Gly Leu Met Tyr Tyr Asn Ser
   305 310 315 320
   Thr Gly Asn Met Gly Val Leu Ala Gly Gln Ala Ser Gln Leu Asn Ala
   325 330 335
   Val Val Asp Leu Gln Asp Arg Asn Thr Glu Leu Ser Tyr Gln Leu Met
   340 345 350
   Leu Asp Ala Leu Gly Asp Arg Ser Arg Tyr Phe Ser Met Trp Asn Gln
           355                 360                 365
   Ala Val Asp Ser Tyr Asp Pro Asp Val Arg Ile Ile Glu Asn His Gly
       370                 375                 380
   Val Glu Asp Glu Leu Pro Asn Tyr Cys Phe Pro Leu Gly Gly Met Ala
   385                 390                 395                 400
   Val Thr Asp Thr Tyr Ser Pro Ile Lys Val Asn Gly Gly Gly Asn Gly
               405                 410                 415
   Trp Glu Ala Asn Asn Gly Val Phe Thr Glu Arg Gly Val Glu Ile Gly
           420                 425                 430
   Ser Gly Asn Met Phe Ala Met Glu Ile Asn Leu Gln Ala Asn Leu Trp
           435                 440                 445
   Arg Ser Phe Leu Tyr Ser Asn Ile Gly Leu Tyr Leu Pro Asp Ser Leu
       450                 455                 460
   Lys Ile Thr Pro Asp Asn Ile Thr Leu Pro Glu Asn Lys Asn Thr Tyr
   465                 470                 475                 480
   Gln Tyr Met Asn Gly Arg Val Thr Pro Pro Gly Leu Val Asp Thr Tyr
               485                 490                 495
   Val Asn Val Gly Ala Arg Trp Ser Pro Asp Val Met Asp Ser Ile Asn
           500                 505                 510
   Pro Phe Asn His His Arg Asn Ala Gly Leu Arg Tyr Arg Ser Met Leu
           515                 520                 525
   Leu Gly Asn Gly Arg Tyr Val Pro Phe His Ile Gln Val Pro Gln Lys
       530                 535                 540
   Phe Phe Ala Ile Lys Asn Leu Leu Leu Leu Pro Gly Ser Tyr Thr Tyr
   545                 550                 555                 560
   Glu Trp Asn Phe Arg Lys Asp Val Asn Met Ile Leu Gln Ser Ser Leu
               565                 570                 575
   Gly Asn Asp Leu Arg Val Asp Gly Ala Ser Ile Arg Phe Asp Ser Ile
   580 585 590
   Asn Leu Tyr Ala Asn Phe Phe Pro Met Ala His Asn Thr Ala Ser Thr
   595 600 605
   Leu Glu Ala Met Leu Arg Asn Asp Thr Asn Asp Gln Ser Phe Asn Asp
   610 615 620
   Tyr Leu Cys Ala Ala Asn Met Leu Tyr Pro Ile Pro Ala Asn Ala Thr
   625 630 635 640
   Ser Val Pro Ile Ser Ile Pro Ser Arg Asn Trp Ala Ala Phe Arg Gly
   645 650 655
   Trp Ser Phe Thr Arg Leu Lys Thr Lys Glu Thr Pro Ser Leu Gly Ser
   660 665 670
   Gly Phe Asp Pro Tyr Phe Val Tyr Ser Gly Ser Ile Pro Tyr Leu Asp
   675 680 685
   Gly Thr Phe Tyr Leu Asn His Thr Phe Lys Lys Val Ser Ile Met Phe
   690 695 700
   Asp Ser Ser Val Ser Trp Pro Gly Asn Asp Arg Leu Leu Thr Pro Asn
   705 710 715 720
   Glu Phe Glu Ile Lys Arg Ser Val Asp Gly Glu Gly Tyr Asn Val Ala
   725 730 735
   Gln Ser Asn Met Thr Lys Asp Trp Phe Leu Ile Gln Met Leu Ser His
   740 745 750
   Tyr Asn Ile Gly Tyr Gln Gly Phe Tyr Val Pro Glu Asn Tyr Lys Asp
   755 760 765
   Arg Met Tyr Ser Phe Phe Arg Asn Phe Gln Pro Met Ser Arg Gln Ile
   770 775 780
   Val Asp Ser Thr Ala Tyr Thr Asn Tyr Gln Asp Val Lys Leu Pro Tyr
   785 790 795 800
   Gln His Asn Asn Ser Gly Phe Val Gly Tyr Met Gly Pro Thr Met Arg
   805 810 815
   Glu Gly Gln Ala Tyr Pro Ala Asn Tyr Pro Tyr Pro Leu Ile Gly Ala
   820 825 830
   Thr Ala Val Pro Ser Leu Thr Gln Leu Leu Phe Leu Cys Asp Arg Val
   835 840 845
   Met Trp Arg Ile Pro Phe Ser Ser Asn Phe Met Ser Met Gly Ser Leu
   850 855 860
   Thr Asp Leu Gly Gln Asn Met Leu Tyr Ala Asn Ser Ala His Ala Leu
   865 870 875 880
   Asp Met Thr Phe Glu Val Asp Pro Met Asp Gln Pro Thr Leu Leu Tyr
   885 890 895
   Val Leu Phe Glu Val Phe Asp Val Val Arg Ile His Gln Pro His Arg
   900 905 910
   Gly Val Ile Glu Ala Val Tyr Leu Arg Thr Pro Phe Ser Ala Gly Asn
   915 920 925
   Ala Thr Thr
   930
<210> 11
   <211> 25
   <212> PRT
   <213> Human adenovirus type 1 hexon region
<400> 11
   Ala Leu Glu Ile Asn Leu Glu Glu Glu Asp Asp Asp Asn Glu Asp Glu
   1 5 10 15
   Val Asp Glu Gln Ala Glu Gln Gln Lys
   20 25
<210> 12
   <211> 14
   <212> PRT
   <213> Human immunodeficiency virus
<400> 12
   Glu Gln Glu Leu Leu Glu Leu Asp Lys Trp Ala Ser Leu Trp
   1               5                   10
<210> 13
   <211> 20
   <212> DNA
   <213> Artificial
<220>
   <223> primer 5hexl
<400> 13
   gaccgccgtt gttgtaaccc 20
<210> 14
   <211> 45
   <212> DNA
   <213> Artificial
<220>
   <223> primer, lt rev
<400> 14
   gttgtcatcg tccactagtc cctcttcttc taggtttatt tcaag 45
<210> 15
   <211> 29
   <212> DNA
   <213> Artificial
<220>
   <223> primer, rt forward
<400> 15
   ggactagtgg acgatgacaa cgaagacga 29
<210> 16
   <211> 20
   <212> DNA
   <213> Artificial
<220>
   <223> primer, rt rev
<400> 16
   atggtttcat tggggtagtc 20
<210> 17
   <211> 48
   <212> DNA
   <213> Artificial
<220>
   <223> oligomer, 2F5 bot
<400> 17
   ccggaccaca ggctggccca cttgtccagc tccagcagct cctgctcg 48
<210> 18
   <211> 36
   <212> DNA
   <213> Artificial
<220>
   <223> oligomer, CD4 top
<400> 18
   ctaggctgct acggcgtgag cgccaccaag ctgggg 36
<210> 19
   <211> 36
   <212> DNA
   <213> Artificial
<220>
   <223> oligomer CD4 bot
<400> 19
   ctagccccag cttggtggcg ctcacgccgt agcagc 36
<210> 20
   <211> 15
   <212> PRT
   <213> Human adenovirus type 5
<400> 20
   Gly Leu Gly Cys Tyr Gly Val Ser Ala Thr Lys Leu Gly Leu Val
   1               5                   10                  15
<210> 21
   <211> 69
   <212> DNA
   <213> Artificial
<220>
   <223> oligomer, CD8 top
<400> 21
   ctagggacca gcaccggcaa ctacaactac aagtaccgct acctgcgcca cggcaagctg 60
   cgccccggg 69
<210> 22
   <211> 69
   <212> DNA
   <213> Artificial
<220>
   <223> oligomer, CD8 bot
<400> 22
   ctagcccggg gcgcagcttg ccgtggcgca ggtagcggta cttgtagttg tagttgccgg 60
   tgctggtcc 69
<210> 23
   <211> 26
   <212> PRT
   <213> Human adenovirus type 5
<400> 23
   Gly Leu Gly Thr Ser Thr Gly Asn Tyr Asn Tyr Lys Tyr Arg Tyr Leu
   1               5                   10                  15
   Arg His Gly Lys Leu Arg Pro Gly Leu Val
               20                  25
<210> 24
   <211> 20
   <212> DNA
   <213> Artificial
<220>
   <223> primer, 5hex1
<400> 24
   gaccgccgtt gttgtaaccc 20
<210> 25
   <211> 35
   <212> DNA
   <213> Artificial
<220>
   <223> primer, BspSOErev
<400> 25
   cgtgagttcc ggaagtagca gcttcatccc attcg 35
<210> 26
   <211> 34
   <212> DNA
   <213> Artificial
<220>
   <223> primer, BspSOEfwd
<400> 26
   tgctacttcc ggaactcacg tatttgggca ggcg 34
<210> 27
   <211> 20
   <212> DNA
   <213> Artificial
<220>
   <223> primer, 5hex4
<400> 27
   ggaagaaggt ggcgtaaagg 20
<210> 28
   <211> 48
   <212> DNA
   <213> Artificial
<220>
   <223> oligomer, 2F5 top
<400> 28
   ccggcgagca ggagctgctg gagctggaca agtgggccag cctgtggt 48

## Description

### BACKGROUND OF THE INVENTION

The present invention relates to modified adenovirus hexon proteins useful in immunogenic regimens.

Adenovirus is a double-stranded DNA virus with a genome size of about 36 kilobases (kb), which has been widely used for gene transfer applications due to its ability to achieve highly efficient gene transfer in a variety of target tissues and large transgene capacity. Conventionally, E1 genes of adenovirus are deleted and replaced with a transgene cassette consisting of the promoter of choice, cDNA sequence of the gene of interest and a poly A signal, resulting in a replication defective recombinant virus.

Adenoviruses have a characteristic morphology with an icosahedral capsid consisting of three major proteins, hexon (II), penton base (III) and a knobbed fibre (IV), along with a number of other minor proteins, VI, VIII, IX, IIIa and IVa2 [W.C. Russell, J. Gen Virol., 81:2573-2604 (Nov 2000)]. The virus genome is a linear, double-stranded DNA with a terminal protein attached covalently to the 5' termini, which have inverted terminal repeats (ITRs). The virus DNA is intimately associated with the highly basic protein VII and a small peptide termed mu. Another protein, V, is packaged with this DNA-protein complex and provides a structural link to the capsid via protein VI. The virus also contains a virus-encoded protease, which is necessary for processing of some of the structural proteins to produce mature infectious virus.

The use of adenoviruses for gene delivery and vaccine regimens has been described. Recombinant adenoviruses have been described for delivery of molecules to host cells. See, US Patent 6,083,716, which describes the genome of two chimpanzee adenoviruses, C1 and C68 (Pan9). See, also, International Patent Publication No. WO 02/33645, which describes vectors constructed from the genomes of chimpanzee adenoviruses SAd22/Pan5, Pan6, Pan 7, as well as simian adenoviruses SV1, SV25 and SV39; and International Patent Publication No. WO 04/16614, which describes hybrid adenovirus vectors and vectors constructed from simian adenovirus SA18.

A variety of modifications to the adenovirus have previously been proposed. Such modifications include insertions into the adenovirus fiber protein [Curiel, D.T., Ann N Y Acad Sci., 886:158-71 (1999)]; insertions in the penton protein for targeting [Einfeld, D.A. et al., J Virol. 73:9130-6) (1999)]; insertions into the adenovirus protein IX for targeting [Dmitriev, I.P. et al., (2002) J Virol. 76:6893-9 (2002)] and modifications of the adenovirus hexon for targeting [Vigne, E. et al., J Virol. 73:5156-61 (1999); US Published Patent Application No. US2003143209 by Latta, Martine, *et al.*] as well as for insertion of an antigenic epitope.

More particularly, insertion of foreign peptides into the hexon protein of human adenovirus serotype 2 (HAdV-2) was reported by Crompton et al., [(1994) J Gen Virol. 75:133-9 (1994)]. With reference to the structure of HAdV-2 reported by Roberts *et al.* [Roberts et al, (1986), Science. 232:1148-51], the authors replaced 17 amino acids of the HAdV-2 hexon (281 - 297) with a 17 amino-acid peptide harboring a poliovirus determinant. The region of the hexon where this substitution was made is now referred to as the DE1 loop between beta sheet elements β₇ and β₈ [Rux JJ, et al., (2003) J Virol. 77:9553-66.]

The work of Crompton et al. (1994) was reproduced by Vigne et al., cited above, in human adenovirus serotype 5 (HAdV-5), which belongs to the same serological subgroup as HAdV-2 and is closely related to it. More particularly, Vigne et al., inserted peptides into the hexon of HAdV-5 at the location identical to that used by Crompton et al. (based on a protein alignment of the HAdV-2 hexon with the HAdV-5 hexon). Vigne et al. replaced 14 HAdV-5 hexon residues (269 - 281) with a poliovirus peptide as well as an integrin-targeting ligand.

Adenovirus hexon hypervariable regions have been described as regions in which sequences differ considerably between serotype (Crawford-Miksza and Schnurr DP. Analysis of 15 adenovirus hexon proteins reveals the location and structure of seven hypervariable regions containing serotype-specific residues J Virol. 1996 70:1836-44; Rux et al., 2003).

WO 2005/026337 describes an adenoviral vector provided with a tropism for Car cells and used for gene transfer. The vector comprises at least one weakly strained structural area of a capsid protein and a heterologous peptide provided with a unit (W/R) X₁X₂D. The vector is used for treating cancers, muscle diseases and mucoviscidosis.

What are needed are alternative methods of delivering molecules to host cells.

### SUMMARY OF THE INVENTION

In one aspect, the invention provides an adenovirus having a capsid comprising a modified adenovirus hexon protein, said modified adenovirus hexon protein comprising a deletion in at least a hypervariable region of an adenovirus hexon protein selected from hypervariable region 1 and/or hypervariable region 4 and an exogenous amino acid sequence comprising an immunogenic amino acid sequence inserted in said hypervariable region, with the proviso that said exogenous amino acid sequence is other than an adenovirus sequence.

In another aspect, the invention provides a method of altering the specificity of an adenovirus vector, the method comprising the step of providing an adenovirus as described above, the modified adenovirus hexon protein of said adenovirus comprising a targeting sequence inserted in the deletion in the hypervariable region.

The invention makes it possible to enhance the immune response to an immunogenic molecule delivered by an adenoviral vector by providing to a subject an adenovirus having a capsid with a modified adenovirus hexon protein, as described above, said adenovirus further containing a nucleic acid molecule encoding an immunogenic molecule packaged in the capsid.

In still another aspect, the invention provides a self-priming immunogenic composition comprising: an adenovirus having a capsid comprising a modified adenovirus hexon protein, as described above, wherein said exogenous amino acid sequence is a first amino acid sequence; wherein said adenovirus further comprises a nucleic acid sequence encoding a second immunogenic amino acid sequence.

Other aspects and advantages of the invention will be readily apparent from the following detailed description of the invention.

### BRIEF DESCRIPTION OF THE FIGURES

Fig. 1 is a map of plasmid pNEBAsc, which results from subcloning the human adenovirus type 5 genome (HAdV-5) harboring the hexon coding region into the plasmid pNEB 193 (purchased from New England Biolabs).

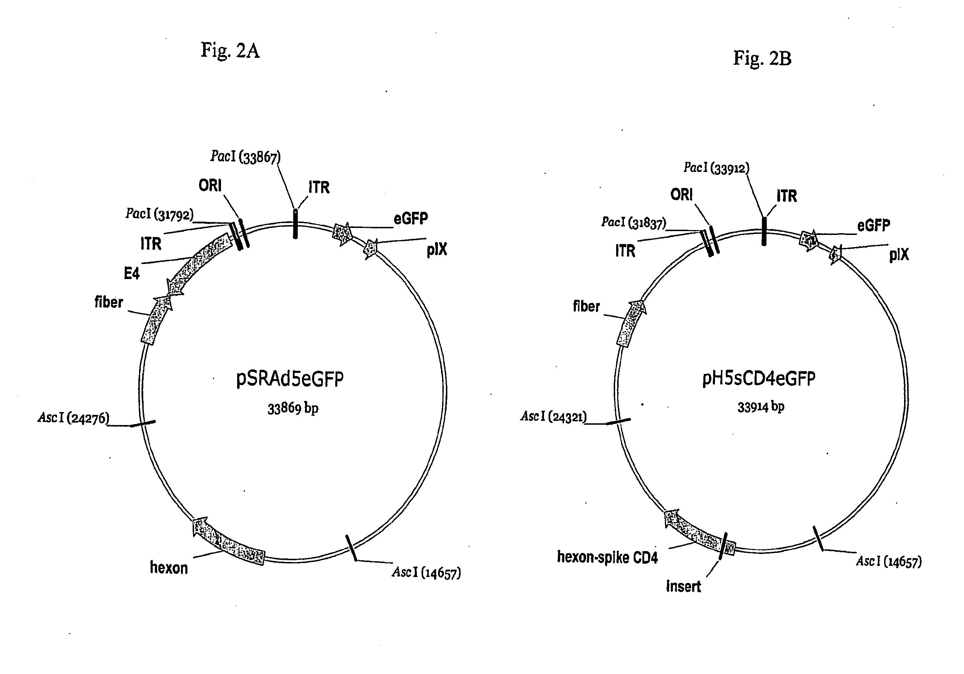

Fig. 2A is a map of plasmid (p) SRAd5eGFP, which is a molecular clone of an E1 and E3-deleted HAdV-5 vector containing the native adenovirus hexon and the green fluorescent protein marker gene.
Fig. 2B is a map of pH5sCD4eGFP, which is a plasmid molecule clone containing the HAdV-5 backbone and the mutated hexon containing the CD4 epitope from the spike protein of the SARS coronavirus.

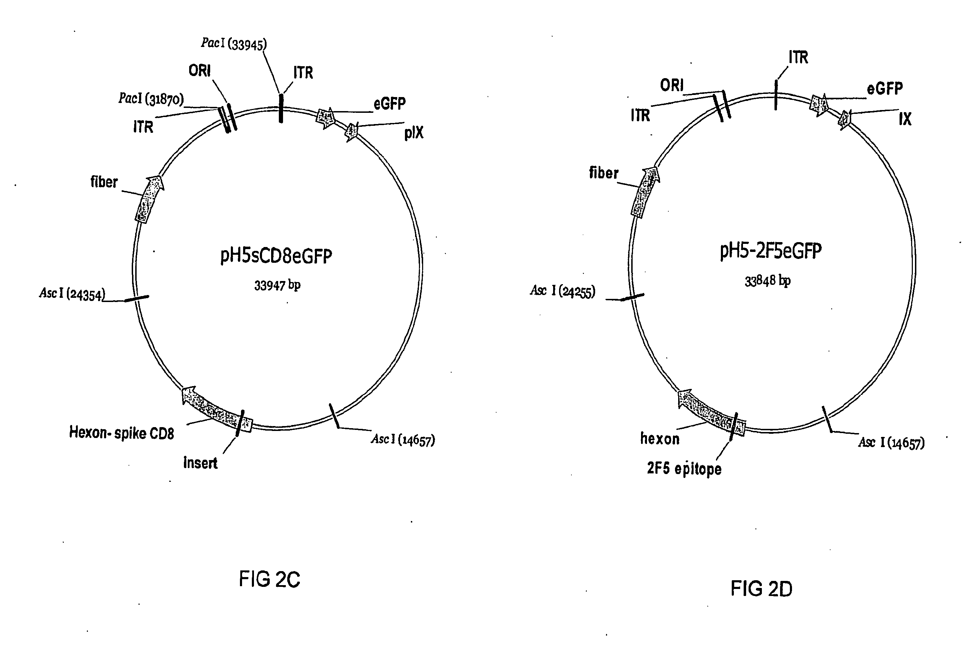

Fig. 2C is a map ofpH5sCD8eGFP, which is a plasmid molecule clone containing the E1, E3-deleted HAdV-5 genome and the mutated hexon containing the CD8 epitope from the spike protein of the SARS coronavirus.
Fig. 2D is a map of pH5-2F5eGFP, which is a plasmid molecule clone containing the E1, E3-deleted HAdV-5 genome and the mutated hexon containing the HIV 2F5 epitope region.

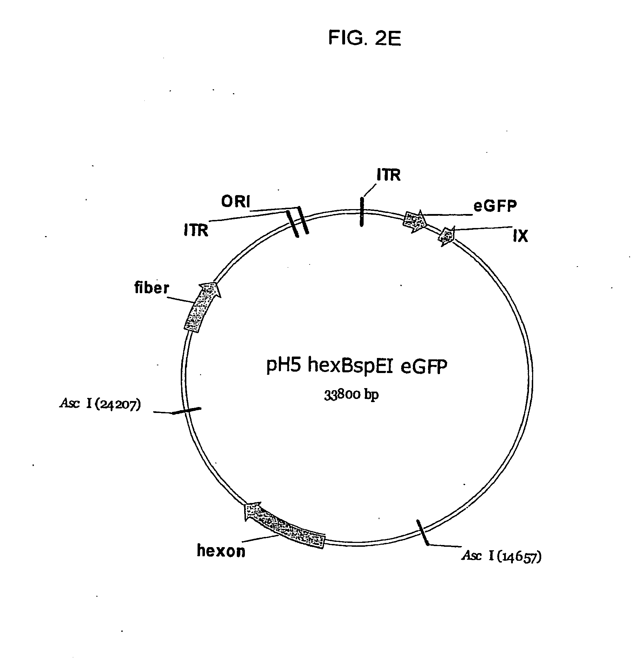

Fig. 2E is a map of pH5-hexBspeEI, which is a plasmid molecule clone containing the E1, E3-deleted HAdV-5 genome and the mutated hexon containing no insert.

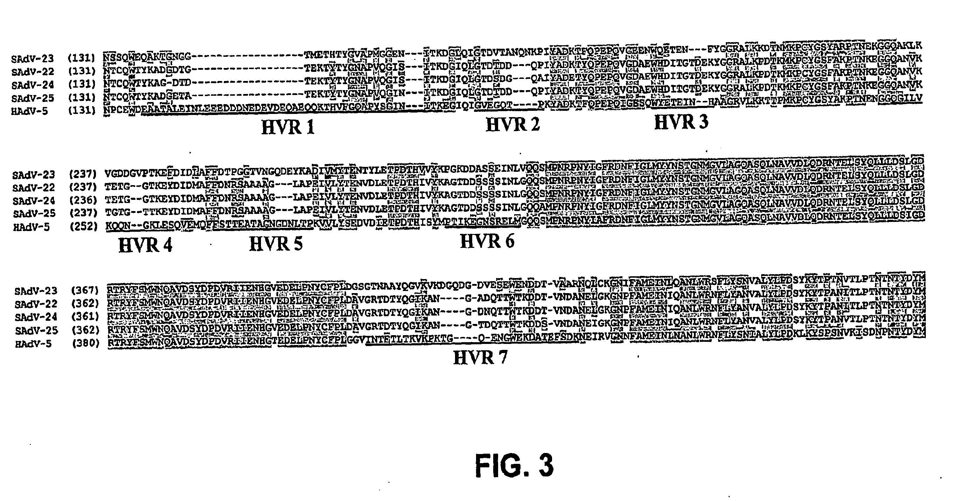

Fig. 3 is an alignment of the portion of the adenovirus hexon protein containing the hypervariable regions (HVR) 1, 2, 3, 4, 5, 6 and 7 for simian adenovirus SAdV-23 (under the old nomenclature, Pan6 or C6); SAdV-22 (under the old nomenclature, SAd22/Pan5 or C5); SAdV-24 (under the old nomenclature Pan7 or C7); and SAdV-25 (under the old nomenclature, C68). The location of the HVR is shown for these sequences, with the numbering relative to each of the sequences shown. For SAdV-23, the fragment is amino acid 131 to 495 of SEQ ID NO: 3; for SAdV-22, the fragment is amino acid 131 to 486 of SEQ ID NO: 4; for SAd24, the fragment is amino acid 131 to 485 of SEQ ID NO: 5; for SAd25, the fragment is amino acid 131 to 486 of SEQ ID NO: 6; for hAd5, the fragment is amino acid 131 to 509 of SEQ ID NO: 2.

### DETAILED DESCRIPTION OF THE INVENTION

The present invention provides a modified adenovirus hexon protein. Such a modified adenovirus hexon protein has a partial or complete deletion in at least one hypervariable region selected from HVR1 and HVR4. The modified adenovirus hexon protein can have one or more independently selected exogenous molecule(s) inserted therein. In one embodiment, an exogenous molecule is inserted in the site of at least the hypervariable region 1 or hypervariable region 4 deletions, with the proviso that the exogenous molecule is not derived from an adenovirus. Alternatively, a modified adenovirus hexon protein can lack any insert in the region of a specified deletion.

A modified adenovirus hexon protein can be used to generate an adenovirus capsid and/or an infectious adenovirus particle. Thus, a modified adenoviral capsid as used herein refers to an adenovirus capsid which contains a modified adenovirus hexon protein as described herein.

In one embodiment, a modified adenovirus capsid of the invention may be empty, *i*.*e*., it lacks a viral genome and/or contains no expression cassette. Such a modified adenoviral capsid can be formulated in a composition for delivery in protein form. Alternatively, such a modified adenoviral capsid can be formulated in a composition for delivery by a suitable vector for expression in a host cell.

In another embodiment, a modified adenoviral capsid is produced in the form of an adenoviral particle having a modified adenovirus capsid having packaged therein one or more heterologous molecules for delivery to a cell. Except where otherwise specified, modified adenoviral vectors as used herein refers to such adenoviral particles.

Hypervariable region 1 (HVR1) and hypervariable region 4 (HVR4) have been identified in the human subgroup C adenoviruses. The structure of these regions is not defined, as are the structures of the other hypervariable regions in the adenovirus subgroup C genome, *i.e.,* HVR2, HVR3, HVR5, HVR6 and HVR7. The hexon protein of human adenovirus serotype 2 is reproduced in SEQ ID NO: for convenience. The hexon protein of human adenovirus serotype 5 is reproduced in SEQ ID NO: 2 for convenience.

Without wishing to be bound by theory, the inventors believe that the modified adenovirus hexon proteins of the invention are advantageous because these HVR regions of the hexon vary significantly in length and sequence between different adenoviruses, thereby providing a flexible construct having tolerance for insertion of a variety of molecules of different lengths. Thus, the invention provides a highly flexible construct which permits insertion of a variety of heterologous molecules useful for targeting and for enhancing immune responses.

The term "functional" refers to a product (*e*.*g*., a protein or peptide) which performs its native function, although not necessarily at the same level as the native product. The term "functional" may also refer to a gene which encodes a product and from which a desired product can be expressed. A "functional deletion" refers to a deletion which destroys the ability of the product to perform its native function.

As used herein, the deletions described herein for the HVR can involve elimination of all or part of the amino acid sequences making up the HVR in the hexon protein. For example, for a HVR of 45 amino acids (*e.g*., HVR1 of Ad2 or Ad5), a deletion of from 1 to 45 amino acids may be made, *e.g*., from 1 to 5, at least 10, at least 25, at least 30, at least 35, or more amino acids may be made. In one embodiment, at least one amino acid from the N-terminus and at least one amino acid from the C-terminus of the native hypervariable region are retained.

As used throughout this specification and the claims, the terms "comprise" and "contain" and its variants including, "comprises", "comprising", "contains" and "containing", among other variants, is inclusive of other components, elements, integers, steps and the like. The term "consists of" or "consisting of" are exclusive of other components, elements, integers, steps and the like.

In one embodiment, a modified adenovirus has a capsid containing a deletion in HVR1 and/or HVR4. In another embodiment, a modified adenovirus has a capsid containing a modification in one or both of these regions and also in another selected HVR, *e.g.,* HVR2, HVR3, HVR5, HVR6 or HVR7.

Hypervariable region 1 is located within residues 139 to 167 or residues 147 through 162 of the hexon protein in HAdV-5 [SEQ ID NO: 2] and HAdV-2 [SEQ ID NO:1], based on the residue numbering of HAdV-2 [SEQ ID NO:1, R. J. Roberts et al, A consensus sequence for the adenovirus-2 genome, p. 1-51, in W. Doerfler (ed.) Adenovirus DNA. Martinus Nijhoff Publishing, Boston.] Hypervariable region 4 is located within residues 222 through 271 of the hexon protein of HAdV-2, based on the same numbering scheme. *See, e.g.,* Fig. 3 for an exemplary alignment providing the HVR locations of other adenoviruses relative to their own numbering. The preparation of such alignments is well within the skill of the art.

Given this information, one of skill in the art can readily determine the corresponding hypervariable regions utilizing alignments of the hexon sequences of different adenovirus sequences, including those from different serotypes within human subgroup C adenoviruses or those from serotypes outside subgroup C adenoviruses. *See, e.g.,* Crawford-Miksza and Schnurr, cited above, and Rux JJ, *et al.,* (2003), cited above, for an illustrative alignment of the sequences of several adenoviruses and the location of the hypervariable regions thereof. Other suitable adenovirus sequences may be readily selected from amongst other human and non-human sequences, which have been described.

As described herein, alignments are performed using any of a variety of publicly or commercially available Multiple Sequence Alignment Programs, such as "Clustal W", accessible through Web Servers on the internet. Alternatively, Vector NTI utilities are also used. There are also a number of algorithms known in the art that can be used to measure nucleotide sequence identity, including those contained in the programs described above. As another example, polynucleotide sequences can be compared using Fasta, a program in GCG Version 6.1. Fasta provides alignments and percent sequence identity of the regions of the best overlap between the query and search sequences. For instance, percent sequence identity between nucleic acid sequences can be determined using Fasta with its default parameters (a word size of 6 and the NOPAM factor for the scoring matrix) as provided in GCG Version 6.1. Similarly programs are available for performing amino acid alignments. Generally, these programs are used at default settings, although one of skill in the art can alter these settings as needed. Alternatively, one of skill in the art can utilize another algorithm or computer program that provides at least the level of identity or alignment as that provided by the referenced algorithms and programs.

Suitable adenoviruses are available from the American Type Culture Collection, Manassas, Virginia, US (ATCC), a variety of academic and commercial sources, or the desired regions may be synthesized using known techniques with reference to sequences published in the literature or available from databases (*e.g*., GenBank, etc.). Examples of suitable adenoviruses include, without limitation, human adenovirus serotypes 2 [sequence published in R. J. Roberts et al, A consensus sequence for the adenovirus-2 genome, p. 1-51, in W. Doerfler (ed.) Adenovirus DNA. Martinus Nijhoff Publishing, Boston., type 3, type 4 [sequences for HAdV-4 genome reported in S. Jacobs et al, J Gen Virol 85 (2004), 3361-3366, reported at GenBank/EMBL/DDBJ accession number AY487947], type 5 [sequence published in R. Kinlock et al, 1984. Adenovirus hexon: sequence comparison of subgroup C serotypes 2 and 5. J. Biol. Chem. 259:6431-6436], AV1 and AV 6 [P. Pring-Akerblom and T. Adrian, 1993, Res. Virol, 144:117-121],7,12 [sequence published in J. Sprengel et al, J Virol, 68:379-389 (1994)], 40 [sequence published in C I Toogood et al, J Gen Virol, 70:3203-3214 (1989)] and further including any of the presently identified human types [see, *e.g*., Horwitz, "Adenoviridae and Their Replication", in VIROLOGY, 2d ed., pp. 1679-1721 (1990)] which can be cultured in the desired cell. Similarly adenoviruses known to infect non-human primates (*e.g*., chimpanzees, rhesus, macaque, and other simian species) or other non-human mammals and which grow in the desired cell can be employed in the vector constructs of this invention. Such serotypes include, without limitation, chimpanzee adenoviruses SAd22/Pan5 [VR-591], SAd23/Pan6 [VR-592], Sad24/Pan7 [VR-593], and SAd25/C68 [Pan9, GenBank accession no. AF394196) and US Patent No. 6,083,716]; and simian adenoviruses including, without limitation SV1 [VR-195]; SV25 [SV-201]; SV35; SV15; SV-34; SV-36; SV-37, and baboon adenovirus [VR-275], among others. The sequences of SAd22/Pan5 (also termed C5), SAd23/Pan 6 (also termed C6), SAd24/Pan 7 (also termed C7), SV1, SV25, and SV39 have been described [WO 03/046124, published 5 June 2003, also published as US-2005-0069866-A1, March 31, 2005].

See, also, International Patent Publication No. WO 04/16614, which describes hybrid adenovirus vectors and vectors constructed from simian adenovirus SA18. The sequences of the hexon proteins of SAd22/Pan5 [SEQ NO: 4], SAd23/Pan6 [SEQ ID NO: 3], SAd24/Pan7 [SEQ ID NO: 5], SV1 [SEQ ID NO: 10], SV25 [SEQ ID NO: 7], SV39 [SEQ ID NO: 8] and SA18 [SEQ ID NO: 9] are reproduced herein. However, one of skill in the art will understand that comparable regions derived from other adenoviral strains may be readily selected and used in the present invention in the place of (or in combination with) these serotypes.

Using these techniques, hypervariable regions in other adenovirus sequences which are analogous to the HVR1 and/or HVR4, in adenovirus subgroup C may also be identified. The modified adenovirus hexons of the invention contain, in addition to these modifications, modifications to one or more of the other hypervariable (HVR) regions.

In one embodiment, a functional deletion is made in a selected HVR and no molecule is inserted therein. This may be desired for a variety of reasons, *e.g*., to accommodate a large insert in another HVR or elsewhere in the capsid. Such a functionally deleted adenovirus hexon protein can be utilized to produce an adenoviral particle, as well as for other uses.

In another embodiment, the modified adenovirus hexon protein contains an exogenous molecule inserted in the deletion in the HVR region. Such an exogenous molecule may be useful to alter the target of the modified adenovirus hexon protein (or a capsid containing same in the form of a viral particle or empty), to alter the immunogenicity of the modified adenovirus capsid, and/or to induce an immune response to the exogenous amino acid sequence or an immunologically cross-reactive molecule.

In one embodiment, the exogenous molecule inserted in the adenovirus hexon protein is an exogenous amino acid sequence of at least two, at least four, at least eight, at least ten, at least fifteen, at least twenty, at least twenty-five, or more amino acid residues in length, or longer, the complete sequence of which is not found in a functionally equivalent position in the amino acid sequence of the wild-type adenovirus, or more particularly in the wild-type viral component protein to be engineered. In certain embodiments, the exogenous amino acid sequence may also be non-native in the sense of not occurring in nature, *i.e.,* being a synthetically or artificially designed or prepared polypeptide. It will be understood that, in the context of a nucleic acid molecule encoding the hexon protein, the exogenous molecule can be in the form of a nucleic acid sequence encoding a desired amino acid sequence.

In one embodiment, the exogenous molecule is an amino acid sequence useful for targeting. As used herein, a "targeting sequence" is a specific amino acid sequence that acts as a signal to direct the modified adenovirus. Thus, in one embodiment, an adenovirus capsid may be modified to bind to a desired target cell to which the native (wild-type) virus from which it is derived does not bind, or it may be modified to have a more restricted binding specificity than the wild-type virus, in other words, to bind to only a selected or particular sub-set or type of target cell from among a broader population of target cell types to which the wild-type virus binds. In another alternative, the adenovirus retains some ability to bind to its original target and an additional target is provided by the exogenous amino acid sequence. The tropism of the virus is thus altered. Hence, by "altered tropism" it is meant that the modified virus exhibits a target cell binding specificity which is altered, or different, to that of the wild-type virus from which it is derived.

Examples of useful exogenous molecules may include, *e.g.,* positively charged amino acids (*e.g*., lysine residues or cysteine residues), cytokines such as interferons and interleukins; lymphokines; membrane receptors such as the receptors recognized by pathogenic organisms (viruses, bacteria or parasites), preferably by the HIV virus (human immunodeficiency virus); coagulation factors such as factor VIII and factor IX; dystrophins; insulin; proteins participating directly or indirectly in cellular ion channels, such as the CFTR (cystic fibrosis transmembrane conductance regulator) protein; antisense RNAs, or proteins capable of inhibiting the activity of a protein produced by a pathogenic gene which is present in the genome of a pathogenic organism, or proteins (or genes encoding them) capable of inhibiting the activity of a cellular gene whose expression is deregulated, for example an oncogene; a protein inhibiting an enzyme activity, such as α1-antitrypsin or a viral protease inhibitor, for example; variants of pathogenic proteins which have been mutated so as to impair their biological function, such as, for example, trans-dominant variants of the tat protein of the HIV virus which are capable of competing with the natural protein for binding to the target sequence, thereby preventing the activation of HIV; antigenic epitopes in order to increase the host cell's immunity; major histocompatibility complex classes I and II proteins, as well as the proteins which are inducers of these genes; antibodies; genes coding for immunotoxins; toxins; growth factors or growth hormones; cell receptors and their ligands; tumor suppressors; proteins involved in cardiovascular disease including, but not limited to, oncogenes; growth factors including, but not limited to, fibroblast growth factor (FGF), vascular endothelial growth factor (VEGF), and nerve growth factor (NGF); e-nos, tumor suppressor genes including, but not limited to, the Rb (retinoblastoma) gene; lipoprotein lipase; superoxide dismutase (SOD); catalase; oxygen and free radical scavengers; apolipoproteins; and pai-1 (plasminogen activator inhibitor-1); cellular enzymes or those produced by pathogenic organisms; suicide genes; hormones, T cell receptors (epitopes antibody epitopes, affibodies and ligands identified from various protein libraries.

In one embodiment, a ligand for a cell surface receptor is the exogenous molecule. Examples of suitable ligands include, *e.g*., antibodies, or antibody fragments or derivatives, single chain antibodies (ScFv), single domain antibodies, and minimal recognition units of antibodies such as a complementary-determining regions (CDRs) of Fv fragments. Such an amino acid sequence may be obtained or derived from the antigen-binding site or antigen binding or recognition/region(s) of an antibody, and such an antibody may be natural or synthetic.

In another embodiment, a viral protein is the exogenous molecule. For example, the HSV-1 TK enzyme has greater affinity compared to the cellular TK enzyme for certain nucleoside analogues (such as acyclovir or gancyclovir).

In still another embodiment, the exogenous molecule is an immunogen. "Immunogenic" molecules may include those which induce a cellular immune response, an antibody response, or both. Vaccinal molecules including those which induce an immune response which is protective against further infection with the pathogen and/or protective against the symptoms of the disease or other condition. An antigen refers to a molecule which is capable of inducing a humoral (antibody) immune response.

Typically, immunogenic molecules induce a specific immune response to a specific virus, organism (*e.g*., bacteria, fungus, yeast), or other source (*e.g*., a tumor) or to a cross-reactive organism or source. However, in certain embodiments, it may be desirable to utilize immunogenic molecules which induce a non-specific immunomodulatory response, *e.g*., by boosting an immune response. For example, such a molecule could be used as a prime in a vaccine regimen or as an adjuvant, depending upon whether it is delivered prior to or together with, a molecule against which an immune response is desired.

Suitable immunogens include, for example, a T-cell epitope (*e.g*., CD4 or CD8 epitope), an antibody epitope, or a peptide, polypeptide, enzyme, or other fragment derived from bacteria, fungi, yeast, and/or viruses. Suitable sources of immunogens include, *inter alia*, the products of the immunogenic transgenes described herein. Still other immunogens will be readily apparent to one of skill in the art.

Thus, in one aspect, the invention provides a modified adenovirus hexon protein, which has been modified to contain one or more exogenous molecules. Where more than one exogenous molecule is utilized, the molecules may be the same. In another embodiment, the exogenous molecules may differ. For example, a modified adenovirus hexon protein may contain an exogenous molecule in one region which modifies native targeting and an exogenous molecule in another region which modifies native neutralizing epitopes and/or induces immune response. In another example, a modified adenovirus hexon protein may contain more than one type of exogenous immunogen. Many other combinations of suitable exogenous molecules, which may be selected independently, will be apparent to one of skill in the art. As will be readily apparent to one of skill in the art, certain molecules may function as both targeting molecules and immunogens.

Techniques for preparing such exogenous molecules (*e.g*., amino acid sequences) and introducing them into viruses or viral components are well known in the art and widely described in the literature. Thus, for example, molecular biology or genetic engineering techniques are readily available, to prepare or construct genetic sequences capable of being expressed as a modified virus, or viral component, according to the present invention. For example, a nucleic acid molecule or nucleotide sequence encoding a viral component protein, may be modified so as to introduce a nucleotide sequence encoding the exogenous amino acid sequence, for example so as to encode a fusion protein comprising all or part of an adenoviral protein and the exogenous amino acid sequence.

It may be convenient or necessary for any such additional or external motif or feature to be incorporated into the virus by means of a "linker" sequence. Such construction techniques for incorporation of DNA or amino acid sequences, via attachment to a linker sequence, are known in the art and are within the routine skill of a protein/genetic engineer.

In another embodiment, the invention utilizes the modified adenovirus capsid containing the exogenous molecule for production of an infectious adenovirus particle, which may contain an expression cassette carrying a desired molecule. Suitably, the molecule carried by the expression cassette encodes a product useful for inducing an immune response to the product or a molecule cross-reactive thereto.

In one example, this embodiment permits the modified adenovirus capsid to function an adjuvant for the molecule delivered by the expression cassette. In another example, this embodiment permits the modified adenovirus capsid to function as a self-priming construct for the molecule delivered by the expression cassette.

### Modified Adenoviral Vectors

In one embodiment, the invention provides a composition comprising a modified adenoviral capsid.

As used herein, a modified adenoviral capsid refers to an adenovirus capsid which contains an adenovirus hexon protein modified as described herein. In addition, a modified adenovirus capsid may contain other modifications. Such other modifications may include other modifications in the hexon protein, or other capsid proteins, *e.g*., the fiber protein or the penton. For example, a modified adenovirus hexon may further contain hexon proteins altered as described in US Patent 5,922,315. In this method, at least one loop region of the adenovirus hexon is changed with at least one loop region of another adenovirus serotype. Still other suitable modifications are described [US Published Patent Application No. 20040171807, published September 2, 2004.] Alternatively or additionally, the modified capsid may be associated with another molecule, e.g., a lipid, a fusion protein.

In one aspect, the compositions of this invention include modified adenoviral vectors. Except where otherwise specified, modified adenoviral vector as used herein refers to an adenoviral particle having a modified adenovirus capsid and which has packaged in the capsid an expression cassette that delivers one or more heterologous molecules to a cells. The adenovirus components utilized in an adenoviral vector can be obtained or derived from a variety of different adenoviruses, such have been described herein and those which are available to one of skill in the art. Because the adenoviral genome contains open reading frames on both strands, in many instances reference is made herein to 5' and 3' ends of the various regions to avoid confusion between specific open reading frames and gene regions. Thus, when reference is made herein to the "left" and "right" end of the adenoviral genome, this reference is to the ends of the approximately 36 kb adenoviral genome when depicted in schematic form as is conventional in the art [*see, e.g*., Horwitz, "Adenoviridae and Their Replication", in VIROLOGY, 2d ed., pp. 1679-1721 (1990)]. Thus, as used herein, the "left terminal end" of the adenoviral genome refers to portion of the adenoviral genome which, when the genome is depicted schematically in linear form, is located at the extreme left end of the schematic. Typically, the left end refers to be portion of the genome beginning at map unit 0 and extending to the right to include at least the 5' inverted terminal repeats (ITRs), and excludes the internal regions of the genome encoding the structural genes. As used herein, the "right terminal end" of the adenoviral genome refers to a portion of the adenoviral genome which, when the genome is depicted schematically in linear form, is located at the extreme right end of the schematic. Typically, the right end of the adenoviral genome refers to a portion of the genome ending at map unit 36 and extending to the left to include at least the 3' ITRs, and excludes the internal regions of the genome encoding the structural genes.

By "minigene" is meant the combination of a selected heterologous gene and the other regulatory elements necessary to drive translation, transcription and/or expression of the gene product in a host cell.

Typically, an adenoviral vector is designed such that the minigene is located in a nucleic acid molecule that contains other adenoviral sequences in the region native to a selected adenoviral gene. The minigene may be inserted into an existing gene region to disrupt the function of that region, if desired. Alternatively, the minigene may be inserted into the site of a partially or fully deleted adenoviral gene. For example, the minigene may be located in the site of such as the site of a functional E1 deletion or functional E3 deletion, among others that may be selected. The term "functionally deleted" or "functional deletion" means that a sufficient amount of the gene region is removed or otherwise damaged, e.g., by mutation or modification, so that the gene region is no longer capable of producing functional products of gene expression. If desired, the entire gene region may be removed. Other suitable sites for gene disruption or deletion are discussed elsewhere in the application.

For example, for a production vector useful for generation of a recombinant virus, the vector may contain the minigene and either the 5' end of the adenoviral genome or the 3' end of the adenoviral genome, or both the 5' and 3' ends of the adenoviral genome. The 5' end of the adenoviral genome contains the 5' cis-elements necessary for packaging and replication; *i.e.,* the 5' inverted terminal repeat (ITR) sequences (which functions as origins of replication) and the native 5' packaging enhancer domains (that contain sequences necessary for packaging linear Ad genomes and enhancer elements for the E1 promoter). The 3' end of the adenoviral genome includes the 3' cis-elements (including the ITRs) necessary for packaging and encapsidation. Suitably, a recombinant adenovirus contains both 5' and 3' adenoviral cis-elements and the minigene is located between the 5' and 3' adenoviral sequences. Any adenoviral vector of the invention may also contain additional adenoviral sequences.

The viral sequences, helper viruses, if needed, and recombinant viral particles, and other vector components and sequences employed in the construction of the vectors described herein are obtained as described above. The DNA sequences of the adenovirus sequences are employed to construct vectors and cell lines useful in the preparation of such vectors.

Modifications of the nucleic acid sequences forming the vectors of this invention, including sequence deletions, insertions, and other mutations may be generated using standard molecular biological techniques and are within the scope of this invention.

The methods employed for the selection of the transgene, the cloning and construction of the "minigene" and its insertion into the viral vector are within the skill in the art given the teachings provided herein.

### 1. The Transgene

The transgene is a nucleic acid sequence, heterologous to the vector sequences flanking the transgene, which encodes a polypeptide, protein, or other product, of interest. The nucleic acid coding sequence is operatively linked to regulatory components in a manner which permits transgene transcription, translation, and/or expression in a host cell. The composition of the transgene sequence will depend upon the use to which the resulting vector will be put. For example, one type of transgene sequence includes a reporter sequence, which upon expression produces a detectable signal. Such reporter sequences include, without limitation, DNA sequences encoding β-lactamase, β-galactosidase (LacZ), alkaline phosphatase, thymidine kinase, green fluorescent protein (GFP), chloramphenicol acetyltransferase (CAT), luciferase, membrane bound proteins including, for example, CD2, CD4, CD8, the influenza hemagglutinin protein, and others well known in the art, to which high affinity antibodies directed thereto exist or can be produced by conventional means, and fusion proteins comprising a membrane bound protein appropriately fused to an antigen tag domain from, among others, hemagglutinin or Myc. These coding sequences, when associated with regulatory elements which drive their expression, provide signals detectable by conventional means, including enzymatic, radiographic, colorimetric, fluorescence or other spectrographic assays, fluorescent activating cell sorting assays and immunological assays, including enzyme linked immunosorbent assay (ELISA), radioimmunoassay (RIA) and immunohistochemistry. For example, where the marker sequence is the LacZ gene, the presence of the vector carrying the signal is detected by assays for beta-galactosidase activity.

Where the transgene is GFP or luciferase, the vector carrying the signal may be measured visually by color or light production in a luminometer. However, desirably, the transgene is a non-marker sequence encoding a product which is useful in biology and medicine, such as protein, peptide, RNA, enzyme, or catalytic RNA. Desirable RNA molecules include tRNA, dsRNA, ribosomal RNA, catalytic RNA, and antisense RNA. One example of a useful RNA sequence is a sequence which extinguishes expression of a targeted nucleic acid sequence in the treated animal.

The transgene may be used for treatment, as a cancer therapeutic or vaccine, for induction of an immune response, and/or for prophylactic vaccine purposes. As used herein, induction of an immune response refers to the ability of a molecule (*e.g*., a gene product) to induce a T cell and/or a humoral immune response to the molecule. The invention further includes using multiple transgenes. In certain situations, a different transgene may be used to encode each subunit of a protein, or to encode different peptides or proteins. This is desirable when the size of the DNA encoding the protein subunit is large, e.g., for an immunoglobulin, the platelet-derived growth factor, or a dystrophin protein. In order for the cell to produce the multi-subunit protein, a cell is infected with the recombinant virus containing each of the different subunits. Alternatively, different subunits of a protein may be encoded by the same transgene. In this case, a single transgene includes the DNA encoding each of the subunits, with the DNA for each subunit separated by an internal ribozyme entry site (IRES). This is desirable when the size of the DNA encoding each of the subunits is small, *e.g.,* the total size of the DNA encoding the subunits and the IRES is less than five kilobases. As an alternative to an IRES, the DNA may be separated by sequences encoding a 2A peptide, which self-cleaves in a post-translational event. See, e.g., M.L. Donnelly, et al, J. Gen. Virol., 78(Pt 1):13-21 (Jan 1997); Furler, S., et al, Gene Ther., 8(11):864-873 (June 2001); Klump H., et al., Gene Ther., 8(10):811-817 (May 2001). This 2A peptide is significantly smaller than an IRES, making it well suited for use when space is a limiting factor. However, the selected transgene may encode any biologically active product or other product, *e.g*., a product desirable for study.

Suitable transgenes may be readily selected by one of skill in the art. The selection of the transgene is not considered to be a limitation of this invention.

### 2. Regulatory Elements

In addition to the major elements identified above for the minigene, the vector also includes conventional control elements necessary which are operably linked to the transgene in a manner that permits its transcription, translation and/or expression in a cell transfected with the plasmid vector or infected with the virus produced by the invention. As used herein, "operably linked" sequences include both expression control sequences that are contiguous with the gene of interest and expression control sequences that act in *trans* or at a distance to control the gene of interest.

Expression control sequences include appropriate transcription initiation, termination, promoter and enhancer sequences; efficient RNA processing signals such as splicing and polyadenylation (polyA) signals; sequences that stabilize cytoplasmic mRNA; sequences that enhance translation efficiency (*i.e*., Kozak consensus sequence); sequences that enhance protein stability; and when desired, sequences that enhance secretion of the encoded product. A great number of expression control sequences, including promoters which are native, constitutive, regulatable and/or tissue-specific, are known in the art and may be utilized.

Examples of constitutive promoters include, without limitation, the retroviral Rous sarcoma virus (RSV) LTR promoter (optionally with the RSV enhancer), the cytomegalovirus (CMV) promoter (optionally with the CMV enhancer) [see, e.g., Boshart et al, Cell, 41:521-530 (1985)], the SV40 promoter, the dihydrofolate reductase promoter, the β-actin promoter, the phosphoglycerol kinase (PGK) promoter, and the EF1α promoter [Invitrogen].

Regulatable promoters allow control of gene expression and can be induced, activated, repressed or "shut off" by exogenously supplied compounds, environmental factors such as temperature, or the presence of a specific physiological state, *e.g*., acute phase, a particular differentiation state of the cell, or in replicating cells only. Regulatable promoters and regulatable systems are available from a variety of commercial sources, including, without limitation, Invitrogen, Clontech and Ariad. Many other systems have been described and can be readily selected by one of skill in the art. For example, inducible promoters include the zinc-inducible sheep metallothionine (MT) promoter and the dexamethasone (Dex)-inducible mouse mammary tumor virus (MMTV) promoter. Other regulatable systems include the T7 polymerase promoter system [WO 98/10088]; the ecdysone insect promoter [No et al, Proc. Natl. Acad. Sci. USA, 93:3346-3351 (1996)], the tetracycline-repressible system [Gossen et al, Proc. Natl. Acad Sci. USA, 89:5547-5551 (1992)], the tetracycline-inducible system [Gossen et al, Science, 268:1766-1769 (1995), see also Harvey et al, Curr. Opin. Chem. Biol., 2:512-518 (1998)]. Other systems include the FK506 dimer, VP16 or p65 using castradiol, diphenol murislerone, the RU486-inducible system [Wang et al, Nat. Biotech., 15:239-243 (1997) and Wang et al, Gene Ther., 4:432-441 (1997)] and the rapamycin-inducible system [Magari et al, J. Clin. Invest., 100:2865-2872 (1997)]. The effectiveness of some regulatable promoters increases over time. In such cases one can enhance the effectiveness of such systems by inserting multiple repressors in tandem, *e.g*., TetR linked to a TetR by an IRES. Alternatively, one can wait at least 3 days before screening for the desired function. One can enhance expression of desired proteins by known means to enhance the effectiveness of this system. For example, using the Woodchuck Hepatitis Virus Posttranscriptional Regulatory Element (WPRE).

In another embodiment, the native promoter for the transgene will be used. The native promoter may be preferred when it is desired that expression of the transgene should mimic the native expression. The native promoter may be used when expression of the transgene must be regulated temporally or developmentally, or in a tissue-specific manner, or in response to specific transcriptional stimuli. In a further embodiment, other native expression control elements, such as enhancer elements, polyadenylation sites or Kozak consensus sequences may also be used to mimic the native expression.

Another embodiment of the transgene includes a transgene operably linked to a tissue-specific promoter. For instance, if expression in skeletal muscle is desired, a promoter active in muscle should be used. These include the promoters from genes encoding skeletal β-action, myosin light chain 2A, dystrophin, muscle creatine kinase, as well as synthetic muscle promoters with activities higher than naturally occurring promoters (see Li et al., Nat. Biotech., 17:241-245 (1999)). Examples of promoters that are tissue-specific are known for liver (albumin, Miyatake et al., J. Virol., 71:5124-32 (1997); hepatitis B virus core promoter, Sandig et al., Gene Ther., 3:1002-9 (1996); alpha-fetoprotein (AFP), Arbuthnot et al., Hum. Gene Ther., 7:1503-14 (1996)), bone osteocalcin (Stein et al., Mol. Biol. Rep., 24:185-96 (1997)); bone sialoprotein (Chen et al., J. Bone Miner. Res., 11:654-64 (1996)), lymphocytes (CD2, Hansal et al., J. Immunol., 161:1063-8 (1998); immunoglobulin heavy chain; T cell receptor chain), neuronal such as neuron-specific enolase (NSE) promoter (Andersen et al., Cell. Mol. Neurobiol., 13:503-15 (1993)), neurofilament light-chain gene (Piccioli et al., Proc. Natl. Acad Sci. USA, 88:5611-5 (1991)), and the neuron-specific vgf gene (Piccioli et al., Neuron, 15:373-84 (1995)), among others.

Optionally, vectors carrying transgenes encoding therapeutically useful or immunogenic products may also include selectable markers or reporter genes may include sequences encoding geneticin, hygromicin or purimycin resistance, among others. Such selectable reporters or marker genes (preferably located outside the viral genome to be packaged into a viral particle) can be used to signal the presence of the plasmids in bacterial cells, such as ampicillin resistance. Other components of the vector may include an origin of replication. Selection of these and other promoters and vector elements are conventional and many such sequences are available [*see, e.g*., Sambrook et al, and references cited therein]. These vectors are generated using the techniques and sequences provided herein, in conjunction with techniques known to those of skill in the art.

Such techniques include conventional cloning techniques of cDNA such as those described in texts [Sambrook et al, Molecular Cloning: A Laboratory Manual, Cold Spring Harbor Press, Cold Spring Harbor, NY], use of overlapping oligonucleotide sequences of the adenovirus genomes, polymerase chain reaction, and any suitable method which provides the desired nucleotide sequence.

### Production of the Modified Adenoviral Particle

At a minimum, a modified adenoviral vector carrying an expression cassette contains the adenovirus cis-elements necessary for replication and virion encapsidation, which cis-elements flank the heterologous gene. That is, the vector contains the cis-acting 5' inverted terminal repeat (ITR) sequences of the adenoviruses which function as origins of replication), the native 5' packaging/enhancer domains (that contain sequences necessary for packaging linear Ad genomes and enhancer elements for the E1 promoter), the heterologous molecule, and the 5' ITR sequences. See, for example, the techniques described for preparation of a "minimal" human Ad vector in US Patent 6,203,975, can be readily adapted for the recombinant simian adenovirus. Optionally, the modified (*e.g*., recombinant) adenoviruses contain more than the minimal adenovirus sequences defined above.

In one embodiment, the adenoviruses are functionally deleted in the E1 a or E1b genes, and optionally bearing other mutations, e.g., temperature-sensitive mutations or deletions in other genes. In other embodiments, it is desirable to retain an intact E1a and/or E1b region in the recombinant adenoviruses. Such an intact E1 region may be located in its native location in the adenoviral genome or placed in the site of a deletion in the native adenoviral genome (*e.g*., in the E3 region).

In the construction of useful adenovirus vectors for delivery of a gene to the human (or other mammalian) cell, a range of adenovirus nucleic acid sequences can be employed in the vectors. For example, all or a portion of the adenovirus delayed early gene E3 may be eliminated from the adenovirus sequence which forms a part of the recombinant virus. The function of E3 is believed to be irrelevant to the function and production of the recombinant virus particle. Modified adenovirus vectors may also be constructed having a deletion of at least the ORF6 region of the E4 gene; and more desirably because of the redundancy in the function of this region, the entire E4 region. Still another vector of this invention contains a deletion in the delayed early gene E2a. Deletions may also be made in any of the late genes L1 through L5 of the adenovirus genome. Similarly, deletions in the intermediate genes IX and IVa₂ may be useful for some purposes. Other deletions may be made in the other structural or non-structural adenovirus genes. The above discussed deletions may be used individually, *i.e.,* an adenovirus sequence for use in the present invention may contain deletions in only a single region. Alternatively, deletions of entire genes or portions thereof effective to destroy their biological activity may be used in any combination. For example, in one exemplary vector, the adenovirus sequence may have deletions of the E1 genes and the E4 gene, or of the E1, E2a and E3 genes, or of the E1 and E3 genes, or of E1, E2a and E4 genes, with or without deletion of E3, and so on. As discussed above, such deletions may be used in combination with other mutations, such as temperature-sensitive mutations, to achieve a desired result.

An adenoviral vector lacking any essential adenoviral sequences (*e.g*., E1a, E1b, E2a, E2b, E4 ORF6, L1, L2, L3, L4 and L5) may be cultured in the presence of the missing adenoviral gene products which are required for viral infectivity and propagation of an adenoviral particle. These helper functions may be provided by culturing the adenoviral vector in the presence of one or more helper constructs (*e.g*., a plasmid or virus) or a packaging host cell. See, for example, the techniques described for preparation of a "minimal" human Ad vector in International Patent Application WO96/13597, published May 9, 1996.

Regardless of whether the modified adenovirus contains only the minimal Ad sequences, or the entire Ad genome with only functional deletions in the E1 and/or E3 regions, in one embodiment, the modified virus contains a capsid derived from a human or simian adenovirus. Alternatively, in other embodiments, pseudotyped adenoviruses may be used in the methods of the invention. Such pseudotyped adenoviruses utilize adenovirus capsid proteins in which a nucleic acid molecule carrying adenovirus sequences from a different source adenovirus than the source of original adenovirus capsid have been packaged. These adenoviruses may be produced using methods that are known to those of skill in the art.

### 1. Helper Viruses

Thus, depending upon the adenovirus gene content of the viral vectors employed to carry the minigene, a helper adenovirus or non-replicating virus fragment may be necessary to provide sufficient simian adenovirus gene sequences necessary to produce an infective recombinant viral particle containing the minigene. Useful helper viruses contain selected adenovirus gene sequences not present in the adenovirus vector construct and/or not expressed by the packaging cell line in which the vector is transfected. In one embodiment, the helper virus is replication-defective and contains a variety of adenovirus genes in addition to the sequences described above. Such a helper virus is desirably used in combination with an El-expressing cell line.

Helper viruses may also be formed into poly-cation conjugates as described in Wu et al, J. Biol. Chem., 264:16985-16987 (1989); K. J. Fisher and J. M. Wilson, Biochem. J., 299:49 (April 1, 1994). Helper virus may optionally contain a second reporter minigene. A number of such reporter genes are known to the art. The presence of a reporter gene on the helper virus which is different from the transgene on the adenovirus vector allows both the Ad vector and the helper virus to be independently monitored. This second reporter is used to enable separation between the resulting recombinant virus and the helper virus upon purification.

### 2. Complementation Cell Lines

To generate modified adenoviruses (Ad) deleted in any of the genes described above, the function of the deleted gene region, if essential to the replication and infectivity of the virus, must be supplied to the recombinant virus by a helper virus or cell line, i.e., a complementation or packaging cell line. In many circumstances, a cell line expressing the human E1 can be used to transcomplement the Ad vector. This is particularly advantageous because, due to the diversity between the Ad sequences of the invention and the human AdE1 sequences found in currently available packaging cells, the use of the current human E1-containing cells prevents the generation of replication-competent adenoviruses during the replication and production process. However, in certain circumstances, it will be desirable to utilize a cell line which expresses the E1 gene products can be utilized for production of an E1-deleted adenovirus. Such cell lines have been described. See, e.g., US Patent 6,083,716.

If desired, one may utilize the sequences provided herein to generate a packaging cell or cell line that expresses, at a minimum, the adenovirus E1 gene from a suitable parental source, or a transcomplementary adenovirus source, under the transcriptional control of a promoter for expression in a selected parent cell line. Regulatable or constitutive promoters may be employed for this purpose. Examples of such promoters are described in detail elsewhere in this specification. A parent cell is selected for the generation of a novel cell line expressing any desired AdSAd22/Pan5, Pan6, Pan7, SV1, SV25 or SV39 gene. Without limitation, such a parent cell line may be HeLa [ATCC Accession No. CCL 2], A549 [ATCC Accession No. CCL 185], HEK 293, KB [CCL 17], Detroit [e.g., Detroit 510, CCL 72] and WI-38 [CCL 75] cells, among others. These cell lines are all available from the American Type Culture Collection, 10801 University Boulevard, Manassas, Virginia 20110-2209. Other suitable parent cell lines may be obtained from other sources.

Such E1-expressing cell lines are useful in the generation of adenovirus E1 deleted vectors. Additionally, or alternatively, the invention provides cell lines that express one or more simian adenoviral gene products, *e.g*., E1a, E1b, E2a, and/or E4 ORF6, can be constructed using essentially the same procedures for use in the generation of recombinant simian viral vectors. Such cell lines can be utilized to transcomplement adenovirus vectors deleted in the essential genes that encode those products, or to provide helper functions necessary for packaging of a helper-dependent virus (*e.g*., adeno-associated virus). The preparation of a host cell according to this invention involves techniques such as assembly of selected DNA sequences. This assembly may be accomplished utilizing conventional techniques. Such techniques include cDNA and genomic cloning, which are well known and are described in Sambrook et al., cited above, use of overlapping oligonucleotide sequences of the adenovirus genomes, combined with polymerase chain reaction, synthetic methods, and any other suitable methods which provide the desired nucleotide sequence.

In still another alternative, the essential adenoviral gene products are provided in *trans* by the adenoviral vector and/or helper virus. In such an instance, a suitable host cell can be selected from any biological organism, including prokaryotic (e.g., bacterial) cells, and eukaryotic cells, including, insect cells, yeast cells and mammalian cells. Particularly desirable host cells are selected from among any mammalian species, including, without limitation, cells such as A549, WEHI, 3T3, 10T1/2, HEK 293 cells or PERC6 (both of which express functional adenoviral E1) [Fallaux, FJ et al, (1998), Hum Gene Ther, 9:1909-1917], Saos, C2C12, L cells, HT1080, HepG2 and primary fibroblast, hepatocyte and myoblast cells derived from mammals including human, monkey, mouse, rat, rabbit, and hamster. The selection of the mammalian species providing the cells is not a limitation of this invention; nor is the type of mammalian cell, *i.e.,* fibroblast, hepatocyte, tumor cell, etc.

### 3. Assembly of Viral Particle and Transfection of a Cell Line

Generally, when delivering the vector comprising the minigene by transfection, the vector is delivered in an amount from about 5 µg to about 100 µg DNA, and preferably about 10 to about 50 µg DNA to about 1 x 10⁴ cells to about 1 x 10¹³ cells, and preferably about 10⁵ cells. However, the relative amounts of vector DNA to host cells may be adjusted, taking into consideration such factors as the selected vector, the delivery method and the host cells selected.

The vector may be any vector known in the art or disclosed above, including naked DNA, a plasmid, phage, transposon, cosmids, episomes, viruses, etc. Introduction into the host cell of the vector may be achieved by any means known in the art or as disclosed above, including transfection, and infection. One or more of the adenoviral genes may be stably integrated into the genome of the host cell, stably expressed as episomes, or expressed transiently. The gene products may all be expressed transiently, on an episome or stably integrated, or some of the gene products may be expressed stably while others are expressed transiently. Furthermore, the promoters for each of the adenoviral genes may be selected independently from a constitutive promoter, an inducible promoter or a native adenoviral promoter. The promoters may be regulated by a specific physiological state of the organism or cell (*i*.*e*., by the differentiation state or in replicating or quiescent cells) or by exogenously-added factors, for example.

Introduction of the molecules (as plasmids or viruses) into the host cell may also be accomplished using techniques known to the skilled artisan and as discussed throughout the specification. In preferred embodiment, standard transfection techniques are used, *e.g*., CaPO₄ transfection or electroporation.

Assembly of the selected DNA sequences of the adenovirus (as well as the transgene and other vector elements) into various intermediate plasmids, and the use of the plasmids and vectors to produce a recombinant viral particle are all achieved using conventional techniques. Such techniques include conventional cloning techniques of cDNA such as those described in texts [Sambrook et al, cited above], use of overlapping oligonucleotide sequences of the adenovirus genomes, polymerase chain reaction, and any suitable method which provides the desired nucleotide sequence. Standard transfection and co-transfection techniques are employed, *e.g*., CaPO₄ precipitation techniques. Other conventional methods employed include homologous recombination of the viral genomes, plaquing of viruses in agar overlay, methods of measuring signal generation, and the like.

For example, following the construction and assembly of the desired minigene-containing viral vector, the vector is transfected *in vitro* in the presence of a helper virus into the packaging cell line. Homologous recombination occurs between the helper and the vector sequences, which permits the adenovirus-transgene sequences in the vector to be replicated and packaged into virion capsids, resulting in the recombinant viral vector particles. The current method for producing such virus particles is transfection-based. However, the invention is not limited to such methods.

The resulting recombinant modified adenoviruses are useful in transferring a selected transgene to a selected cell.

### Use of the Modified Adenovirus Vectors

The modified adenovirus vectors of the invention are useful for gene transfer to a human or veterinary subject (including, non-human primates, non-simian primates, and other mammals) *in vitro*, *ex vivo*, and *in vivo.*

In one embodiment, the modified adenovirus vectors described herein can be used as expression vectors for the production of the products encoded by the heterologous genes *in vitro.* For example, a suitable cell line is infected or transfected with the modified adenoviruses and cultured in the conventional manner, allowing the modified adenovirus to express the gene product from the promoter. The gene product may then be recovered from the culture medium by known conventional methods of protein isolation and recovery from culture.

In another embodiment, a modified adenoviral vector of the invention provides an efficient gene transfer vehicle that can deliver a selected transgene to a selected host cell *in vivo* or *ex vivo.* For example, the modified adenoviral vectors of the invention can be utilized for delivery of therapeutic or immunogenic molecules, as described below. In one embodiment, a therapeutic or vaccine regimen will involve repeat delivery of recombinant adenoviral vectors. Such regimens typically involve delivery of a series of viral vectors in which the viral capsids are alternated. The viral capsids may be changed for each subsequent administration, or after a pre-selected number of administrations of a particular serotype capsid (*e*.*g*., one, two, three, four or more). Thus, a regimen may involve delivery of an Ad with a first capsid, delivery with an Ad with a second capsid, and delivery with a third capsid. In other embodiments, a regimen may be selected which uses the modified Ad capsids of the invention alone, in combination with one another, or in combination with other Ad serotypes, as will be apparent to those of skill in the art. Optionally, such a regimen may involve administration of Ad with capsids of non-human primate adenoviruses, human adenoviruses, or artificial (*e*.*g.,* chimeric) serotypes such as are described herein. Each phase of the regimen may involve administration of a series of injections (or other delivery routes) with a single Ad serotype capsid followed by a series with another Ad serotype capsid. Alternatively, the modified Ad vectors of the invention may be utilized in regimens involving other non-adenoviral-mediated delivery systems, including other viral systems, non-viral delivery systems, protein, peptides, and other biologically active molecules.

The following sections will focus on exemplary molecules which may be delivered via the modified adenoviral capsids (*e.g*., in protein form) or modified adenoviral vectors of the invention.

In one embodiment, the modified Ad vectors described herein are administered to humans according to published methods for gene therapy. A viral vector of the invention bearing the selected transgene may be administered to a patient, preferably suspended in a biologically compatible solution or pharmaceutically acceptable delivery vehicle. A suitable vehicle includes sterile saline. Other aqueous and non-aqueous isotonic sterile injection solutions and aqueous and non-aqueous sterile suspensions known to be pharmaceutically acceptable carriers and well known to those of skill in the art may be employed for this purpose.

The modified adenoviral vectors are administered in sufficient amounts to provide the desired physiologic effect. Conventional and pharmaceutically acceptable routes of administration include, but are not limited to, direct delivery to the retina and other intraocular delivery methods, direct delivery to the liver, inhalation, intranasal, intravenous, intramuscular, intratracheal, subcutaneous, intradermal, rectal, oral, intraocular, intracochlear, and other parenteral routes of administration. Routes of administration may be combined, if desired, or adjusted depending upon the transgene or the condition. The route of administration primarily will depend on the nature of the condition being treated.

Dosages of the viral vector will depend primarily on factors such as the condition being treated, the age, weight and health of the patient, and may thus vary among patients. For example, a therapeutically effective adult human or veterinary dosage of the viral vector is generally in the range of from about 100 µL to about 100 mL of a carrier containing concentrations of from about 1 x 10⁶ to about 1 x 10¹⁵ particles, about 1 x 10¹¹ to 1 x 10¹³ particles, or about 1 x 10⁹ to 1x 10¹² particles virus. Dosage ranges will depend upon the size of the animal and the route of administration. For example, a suitable human or veterinary dosage (for about an 80 kg animal) for intramuscular injection is in the range of about 1 x 10⁹ to about 5 x 10¹² particles per mL, for a single site. Optionally, multiple sites of administration may be delivered. In another example, a suitable human or veterinary dosage may be in the range of about 1 x 10¹¹ to about 1 x 10¹⁵ particles for an oral formulation. One of skill in the art may adjust these doses, depending the route of administration, and the therapeutic or vaccinal application for which the recombinant vector is employed. The levels of expression of the transgene, or for an immunogen, the level of circulating antibody, can be monitored to determine the frequency of dosage administration. Yet other methods for determining the timing of frequency of administration will be readily apparent to one of skill in the art.

### 1. Therapeutic Molecules

In one aspect, the modified adenovirus hexon proteins can contain inserts of one or more therapeutic transgenes, in order to facilitate targeting and/or for use in a therapeutic regimen as described herein. In another aspect, the modified adenoviral vectors of the invention may carry packaged therein one or therapeutic transgenes.

Useful therapeutic products encoded by the transgene include hormones and growth and differentiation factors including, without limitation, insulin, glucagon, growth hormone (GH), parathyroid hormone (PTH), growth hormone releasing factor (GRF), follicle stimulating hormone (FSH), luteinizing hormone (LH), human chorionic gonadotropin (hCG), vascular endothelial growth factor (VEGF), angiopoietins, angiostatin, granulocyte colony stimulating factor (GCSF), erythropoietin (EPO), connective tissue growth factor (CTGF), basic fibroblast growth factor (bFGF), acidic fibroblast growth factor (aFGF), epidermal growth factor (EGF), transforming growth factor α (TGF α), platelet-derived growth factor (PDGF), insulin growth factors I and II (IGF-I and IGF-II), any one of the transforming growth factor superfamily, including TGF, activins, inhibins, or any of the bone morphogenic proteins (BMP) BMPs 1-15, any one of the heregluin/neuregulin/ARIA/neu differentiation factor (NDF) family of growth factors, nerve growth factor (NGF), brain-derived neurotrophic factor (BDNF), neurotrophins NT-3 and NT-4/5, ciliary neurotrophic factor (CNTF), glial cell line derived neurotrophic factor (GDNF), neurturin, agrin, any one of the family of semaphorins/collapsins, netrin-1 and netrin-2, hepatocyte growth factor (HGF), ephrins, noggin, sonic hedgehog and tyrosine hydroxylase.

Other useful transgene products include proteins that regulate the immune system including, without limitation, cytokines and lymphokines such as thrombopoietin (TPO), interleukins (IL) IL-1 through IL-25 (including, *e.g*., IL-2, IL-4, IL-12 and IL-18), monocyte chemoattractant protein, leukemia inhibitory factor, granulocyte-macrophage colony stimulating factor, Fas ligand, tumor necrosis factors and, interferons, and, stem cell factor, flk-2/flt3 ligand. Gene products produced by the immune system are also useful in the invention. These include, without limitation, immunoglobulins IgG, IgM, IgA, IgD and IgE, chimeric immunoglobulins, humanized antibodies, single chain antibodies, T cell receptors, chimeric T cell receptors, single chain T cell receptors, class I and class II MHC molecules, as well as engineered immunoglobulins and MHC molecules. Useful gene products also include complement regulatory proteins such as complement regulatory proteins, membrane cofactor protein (MCP), decay accelerating factor (DAF), CR1, CF2 and CD59. Still other useful gene products include any one of the receptors for the hormones, growth factors, cytokines, lymphokines, regulatory proteins and immune system proteins. The invention encompasses receptors for cholesterol regulation, including the low density lipoprotein (LDL) receptor, high density lipoprotein (HDL) receptor, the very low density lipoprotein (VLDL) receptor, proteins useful in the regulation of lipids, including, e.g., apolipoprotein (apo) A and its isoforms (*e.g*., ApoAI), apoE and its isoforms including E2, E3 and E4), SRB1, ABC1, and the scavenger receptor. The invention also encompasses gene products such as members of the steroid hormone receptor superfamily including glucocorticoid receptors and estrogen receptors, Vitamin D receptors and other nuclear receptors. In addition, useful gene products include transcription factors such as jun, fos, max, mad, serum response factor (SRF), AP-1, AP2, myb, MyoD and myogenin, ETS-box containing proteins, TFE3, E2F, ATF1, ATF2, ATF3, ATF4, ZF5, NFAT, CREB, HNF-4, C/EBP, SP1, CCAAT-box binding proteins, interferon regulation factor (IRF-1), Wilms tumor protein, ETS-binding protein, STAT, GATA-box binding proteins, e.g., GATA-3, and the forkhead family of winged helix proteins.

Other useful gene products include, carbamoyl synthetase I, ornithine transcarbamylase, arginosuccinate synthetase, arginosuccinate lyase, arginase, fumarylacetacetate hydrolase, phenylalanine hydroxylase, alpha-1 antitrypsin, glucose-6-phosphatase, porphobilinogen deaminase, cystathione beta-synthase, branched chain ketoacid decarboxylase, albumin, isovaleryl-coA dehydrogenase, propionyl CoA carboxylase, methyl malonyl CoA mutase, glutaryl CoA dehydrogenase, insulin, beta-glucosidase, pyruvate carboxylate, hepatic phosphorylase, phosphorylase kinase, glycine decarboxylase, H-protein, T-protein, a cystic fibrosis transmembrane regulator (CFTR) sequence, and a dystrophin cDNA sequence. Other useful gene products include those useful for treatment of hemophilia A (*e*.*g*., Factor VIII and its variants, including the light chain and heavy chain of the heterodimer, optionally operably linked by a junction), and the B-domain deleted Factor VIII, see US Patent Nos. 6,200,560 and 6,221,349], and useful for treatment of hemophilia B (*e.g*., Factor IX).

Still other useful gene products include non-naturally occurring polypeptides, such as chimeric or hybrid polypeptides having a non-naturally occurring amino acid sequence containing insertions, deletions or amino acid substitutions. For example, single-chain engineered immunoglobulins could be useful in certain immunocompromised patients. Other types of non-naturaily occurring gene sequences include antisense molecules and catalytic nucleic acids, such as ribozymes, which could be used to reduce overexpression of a target.

Reduction and/or modulation of expression of a gene are particularly desirable for treatment of hyperproliferative conditions characterized by hyperproliferating cells, as are cancers and psoriasis. Target polypeptides include those polypeptides which are produced exclusively or at higher levels in hyperproliferative cells as compared to normal cells. Target antigens include polypeptides encoded by oncogenes such as myb, myc, fyn, and the translocation gene bcr/abl, ras, src, P53, neu, trk and EGRF. In addition to oncogene products as target antigens, target polypeptides for anti-cancer treatments and protective regimens include variable regions of antibodies made by B cell lymphomas and variable regions ofT cell receptors of T cell lymphomas which, in some embodiments, are also used as target antigens for autoimmune disease. Other tumor-associated polypeptides can be used as target polypeptides such as polypeptides which are found at higher levels in tumor cells including the polypeptide recognized by monoclonal antibody 17-1A and folate binding polypeptides.

Other suitable therapeutic polypeptides and proteins include those which may be useful for treating individuals suffering from autoimmune diseases and disorders by conferring a broad based protective immune response against targets that are associated with autoimmunity including cell receptors and cells which produce self-directed antibodies. T-cell mediated autoimmune diseases include rheumatoid arthritis (RA), multiple sclerosis (MS), Sjögren's syndrome, sarcoidosis, insulin dependent diabetes mellitus (IDDM), autoimmune thyroiditis, reactive arthritis, ankylosing spondylitis, scleroderma, polymyositis, dermatomyositis, psoriasis, vasculitis, Wegener's granulomatosis, Crohn's disease and ulcerative colitis. Each of these diseases is characterized by T cell receptors (TCRs) that bind to endogenous antigens and initiate the inflammatory cascade associated with autoimmune diseases.

The modified adenoviral vectors of the invention are particularly well suited for therapeutic regimens in which multiple adenoviral-mediated deliveries of transgenes is desired, *e.g.,* in regimens involving redelivery of the same transgene or in combination regimens involving delivery of other transgenes. Such regimens may involve administration of a modified adenoviral vector, followed by re-administration with a vector from the same serotype adenovirus. Particularly desirable regimens involve administration of a modified adenoviral vector of the invention, in which the serotype of the viral vector delivered in the first administration differs from the serotype of the viral vector utilized in one or more of the subsequent administrations. For example, a therapeutic regimen involves administration of a modified adenoviral and repeat administration with one or more modified adenoviruses of the same or different serotypes. In another example, a therapeutic regimen involves administration of an adenoviral vector followed by repeat administration with a modified adenovirus vector of the invention which differs from the serotype of the first delivered adenoviral vector, and optionally further administration with another vector which is the same or, preferably, differs from the serotype of the vector in the prior administration steps. These regimens are not limited to delivery of adenoviral vectors constructed using the chimeric serotypes of the invention. Rather, these regimens can readily utilize vectors of other adenoviral serotypes (whether modified or not), which may be of artificial, human or non-human primate, or other mammalian sources, in combination with one or more of the chimeric vectors of the invention. Examples of such serotypes are discussed elsewhere in this document. Further, these therapeutic regimens may involve either simultaneous or sequential delivery of modified adenoviral vectors of the invention in combination with non-adenoviral vectors, non-viral vectors, and/or a variety of other therapeutically useful compounds or molecules. The present invention is not limited to these therapeutic regimens, a variety of which will be readily apparent to one of skill in the art.

### 2. Delivery of Immunogenic Transgenes

As previously described, the modified adenovirus hexon protein itself may be modified to contain a selected a peptide, polypeptide or protein which induces an immune response to a selected immunogen. Such a modified adenovirus hexon protein itself may be used to generate an adenovirus capsid which is itself useful for targeting, as an adjuvant, and/or for inducing a specific immune response. In a further embodiment, such a modified adenovirus capsid is used in the generation of a viral particle, in which a suitable expression cassette or minigene is packaged.

Thus, in one embodiment, a modified adenoviral vector of the invention further contains packaged within the modified adenovirus capsid a transgene encoding a peptide, polypeptide or protein which induces an immune response to a selected immunogen. Such modified adenoviruses of this invention may provide a self-priming effect, an adjuvant effect, and/or may be highly efficacious at inducing cytolytic T cells and/or antibodies to the inserted heterologous protein(s) expressed by the vector.

The adenoviruses of the invention may also be employed as immunogenic compositions. The recombinant adenoviruses can be used as prophylactic or therapeutic vaccines against any pathogen for which the antigen(s) crucial for induction of an immune response and able to limit the spread of the pathogen has been identified and for which the cDNA is available.

Such vaccinal (or other immunogenic) compositions are formulated in a suitable delivery vehicle, as described above. Generally, doses for the immunogenic compositions are in the range defined above for therapeutic compositions. The levels of immunity of the selected gene can be monitored to determine the need, if any, for boosters. Following an assessment of antibody titers in the serum, optional booster immunizations may be desired.

Optionally, a vaccinal composition of the invention may be formulated to contain other components, including, e.g. adjuvants, stabilizers, pH adjusters, preservatives and the like. Such components are well known to those of skill in the vaccine art. Examples of suitable adjuvants include, without limitation, liposomes, alum, monophosphoryl lipid A, and any biologically active factor, such as cytokine, an interleukin, a chemokine, a ligands, and optimally combinations thereof. Certain of these biologically active factors can be expressed *in vivo*, *e*.*g*., via a plasmid or viral vector. For example, such an adjuvant can be administered with a priming DNA vaccine encoding an antigen to enhance the antigen-specific immune response compared with the immune response generated upon priming with a DNA vaccine encoding the antigen only.

The adenoviruses are administered in "an immunogenic amount", that is, an amount of adenovirus that is effective in a route of administration to transfect the desired cells and provide sufficient levels of expression of the selected gene to induce an immune response. Where protective immunity is provided, the adenoviruses are considered to be vaccine compositions useful in preventing infection and/or recurrent disease.

For example, immunogens may be selected from a variety of viral families. Example of desirable viral families against which an immune response would be desirable include, the picornavirus family, which includes the genera rhinoviruses, which are responsible for about 50% of cases of the common cold; the genera enteroviruses, which include polioviruses, coxsackieviruses, echoviruses, and human enteroviruses such as hepatitis A virus; and the genera apthoviruses, which are responsible for foot and mouth diseases, primarily in non-human animals. Within the picornavirus family of viruses, target antigens include the VP1, VP2, VP3, VP4, and VPG. Another viral family includes the calcivirus family, which encompasses the Norwalk group of viruses, which are an important causative agent of epidemic gastroenteritis. Still another viral family desirable for use in targeting antigens for inducing immune responses in humans and non-human animals is the togavirus family, which includes the genera alphavirus, which include Sindbis viruses, RossRiver virus, and Venezuelan, Eastern & Western Equine encephalitis, and rubivirus, including Rubella virus. The flaviviridae family includes dengue, yellow fever, Japanese encephalitis, St. Louis encephalitis and tick borne encephalitis viruses. Other target antigens may be generated from the Hepatitis C or the coronavirus family, which includes a number of non-human viruses such as infectious bronchitis virus (poultry), porcine transmissible gastroenteric virus (pig), porcine hemagglutinatin encephalomyelitis virus (pig), feline infectious peritonitis virus (cats), feline enteric coronavirus (cat), canine coronavirus (dog), and human respiratory coronaviruses, which may cause the common cold and/or non-A, B or C hepatitis. In addition, the human coronaviruses include the putative causative agent of sudden acute respiratory syndrome (SARS). Within the coronavirus family, target antigens include the E1 (also called M or matrix protein), E2 (also called S or Spike protein), E3 (also called HE or hemagglutin-elterose) glycoprotein (not present in all coronaviruses), or N (nucleocapsid). Still other antigens may be targeted against the rhabdovirus family, which includes the genera vesiculovirus (e.g., Vesicular Stomatitis Virus), and the general lyssavirus (*e.g*., rabies). Within the rhabdovirus family, suitable antigens may be derived from the G protein or the N protein. The family filoviridae, which includes hemorrhagic fever viruses such as Marburg and Ebola virus, may be a suitable source of antigens. The paramyxovirus family includes parainfluenza Virus Type 1, parainfluenza Virus Type 3, bovine parainfluenza Virus Type 3, rubulavirus (mumps virus), parainfluenza Virus Type 2, parainfluenza virus Type 4, Newcastle disease virus (chickens), rinderpest, morbillivirus, which includes measles and canine distemper, and pneumovirus, which includes respiratory syncytial virus. The influenza virus is classified within the family orthomyxovirus and is a suitable source of antigen (*e.g*., the HA protein, the N1 protein). The bunyavirus family includes the genera bunyavirus (California encephalitis, La Crosse), phlebovirus (Rift Valley Fever), hantavirus (puremala is a hemahagin fever virus), nairovirus (Nairobi sheep disease) and various unassigned bungaviruses. The arenavirus family provides a source of antigens against LCM and Lassa fever virus. The reovirus family includes the genera reovirus, rotavirus (which causes acute gastroenteritis in children), orbiviruses, and cultivirus (Colorado Tick fever), Lebombo (humans), equine encephalosis, blue tongue. The retrovirus family includes the sub-family oncorivirinal which encompasses such human and veterinary diseases as feline leukemia virus, HTLVI and HTLVII, lentivirinal (which includes human immunodeficiency virus (HIV), simian immunodeficiency virus (SIV), feline immunodeficiency virus (FIV), equine infectious anemia virus, and spumavirinal). Among the lentiviruses, many suitable antigens have been described and can readily be selected.

Examples of suitable HIV and SIV antigens include, without limitation the gag, pol, Vif, Vpx, VPR, Env, Tat, Nef, and Rev proteins, as well as various fragments thereof. For example, suitable fragments of the Env protein may include any of its subunits such as the gp120, gp160, gp41, or smaller fragments thereof, e.g., of at least about 8 amino acids in length. Similarly, fragments of the tat protein may be selected. [See, US Patent 5,891,994 and US Patent 6,193,981.] See, also, the HIV and SIV proteins described in D.H. Barouch et al, J. Virol., 75(5):2462-2467 (March 2001), and R.R. Amara, et al, Science, 292:69-74 (6 April 2001). In another example, the HIV and/or SIV immunogenic proteins or peptides may be used to form fusion proteins or other immunogenic molecules. *See, e.g*., the HIV-1 Tat and/or Nef fusion proteins and immunization regimens described in WO 01/54719, published August 2, 2001, and WO 99/16884, published April 8, 1999. The invention is not limited to the HIV and/or SIV immunogenic proteins or peptides described herein. In addition, a variety of modifications to these proteins have been described or could readily be made by one of skill in the art. See, *e.g.,* the modified gag protein that is described in US Patent 5,972,596. Further, any desired HIV and/or SIV immunogens may be delivered alone or in combination. Such combinations may include expression from a single vector or from multiple vectors. Optionally, another combination may involve delivery of one or more expressed immunogens with delivery of one or more of the immunogens in protein form. Such combinations are discussed in more detail below.

The papovavirus family includes the sub-family polyomaviruses (BKU and JCU viruses) and the sub-family papillomavirus (associated with cancers or malignant progression of papilloma). The adenovirus family includes viruses (EX, AD7, ARD, O.B.) which cause respiratory disease and/or enteritis. The parvovirus includes family feline parvovirus (feline enteritis), feline panleucopeniavirus, canine parvovirus, and porcine parvovirus. The herpesvirus family includes the sub-family alphaherpesvirinae, which encompasses the genera simplexvirus (HSVI, HSVII), varicellovirus (pseudorabies, varicella zoster) and the sub-family betaherpesvirinae, which includes the genera cytomegalovirus (HCMV, muromegalovirus) and the sub-family gammaherpesvirinae, which includes the genera lymphocryptovirus, EBV (Burkitts lymphoma), infectious rhinotracheitis, Marek's disease virus, and rhadinovirus. The poxvirus family includes the sub-family chordopoxvirinae, which encompasses the genera orthopoxvirus (Variola (Smallpox) and Vaccinia (Cowpox)), parapoxvirus, avipoxvirus, capripoxvirus, leporipoxvirus, suipoxvirus, and the sub-family entornopoxvirinae. The hepadnavirus family includes the Hepatitis B virus. One unclassified virus which may be suitable source of antigens is the Hepatitis delta virus. Still other viral sources may include avian infectious bursal disease virus and porcine respiratory and reproductive syndrome virus. The alphavirus family includes equine arteritis virus and various Encephalitis viruses.

The viruses of the present invention may also carry immunogens which are useful to immunize a human or non-human animal against other pathogens including bacteria, fungi, parasitic microorganisms or multicellular parasites which infect human and non-human vertebrates, or from a cancer cell or tumor cell. Examples of bacterial pathogens include pathogenic gram-positive cocci include pneumococci; staphylococci; and streptococci. Pathogenic gram-negative cocci include meningococcus; gonococcus. Pathogenic enteric gram-negative bacilli include enterobacteriaceae; pseudomonas, acinetobacteria and eikenella; melioidosis; salmonella; shigella; haemophilus; moraxella; *H. ducreyi* (which causes chancroid); brucella; *Franisella tularensis* (which causes tularemia); yersinia (pasteurelia); streptobacillus moniliformis and spirillum; Gram-positive bacilli include listeria monocytogenes; erysipelothrix rhusiopathiae; *Corynebacterium diphtheria* (diphtheria); cholera; *B. anthracis* (anthrax); donovanosis (granuloma inguinale); and bartonellosis. Diseases caused by pathogenic anaerobic bacteria include tetanus; botulism; other clostridia; tuberculosis; leprosy; and other mycobacteria. Pathogenic spirochetal diseases include syphilis; treponematoses: yaws, pinta and endemic syphilis; and leptospirosis. Other infections caused by higher pathogen bacteria and pathogenic fungi include actinomycosis; nocardiosis; cryptococcosis, blastomycosis, histoplasmosis and coccidioidomycosis; candidiasis, aspergillosis, and mucormycosis; sporotrichosis; paracoccidiodomycosis, petriellidiosis, torulopsosis, mycetoma arid chromomycosis; and dermatophytosis. Rickettsial infections include Typhus fever, Rocky Mountain spotted fever, Q fever, and Rickettsialpox. Examples of mycoplasma and chlamydial infections include: mycoplasma pneumoniae; lymphogranuloma venereum; psittacosis; and perinatal chlamydial infections. Pathogenic eukaryotes encompass pathogenic protozoans and helminths and infections produced thereby include: amebiasis; malaria; leishmaniasis; trypanosomiasis; toxoplasmosis; *Pneumocystis carinii*; Trichans; *Toxoplasma gondii*; babesiosis; giardiasis; trichinosis; filariasis; schistosomiasis; nematodes; trematodes or flukes; and cestode (tapeworm) infections. Many of these organisms and/or toxins produced thereby have been identified by the Centers for Disease Control [(CDC), Department of Heath and Human Services, USA], as agents which have potential for use in biological attacks. For example, some of these biological agents, include, *Bacillus anthracis* (anthrax), *Clostridium botulinum* and its toxin (botulism), *Yersinia pestis* (plague), variola major (smallpox), *Francisella tularensis* (tularemia), and viral hemorrhagic fevers [filoviruses (e.g., Ebola, Marburg], and arenaviruses [e.g., Lassa, Machupo]), all of which are currently classified as Category A agents; *Coxiella burnetti* (Q fever); Brucella species (brucellosis), *Burkholderia mallei* (glanders), *Burkholderia pseudomallei* (meloidosis), *Ricinus communis* and its toxin (ricin toxin), *Clostridium perfringens* and its toxin (epsilon toxin), Staphylococcus species and their toxins (enterotoxin B), *Chlamydia psittaci* (psittacosis), water safety threats (e.g., *Vibrio cholerae*, *Crytosporidium parvum*), Typhus fever (*Richettsia powazekii*), and viral encephalitis (alphaviruses, *e.g.,* Venezuelan equine encephalitis; eastern equine encephalitis; western equine encephalitis); all of which are currently classified as Category B agents; and Nipan virus and hantaviruses, which are currently classified as Category C agents. In addition, other organisms, which are so classified or differently classified, may be identified and/or used for such a purpose in the future. It will be readily understood that the viral vectors and other constructs described herein are useful to deliver antigens from these organisms, viruses, their toxins or other by-products, which will prevent and/or treat infection or other adverse reactions with these biological agents.

Administration of the vectors of the invention to deliver immunogens against the variable region of the T cells elicits an immune response including cytotoxic lymphocytes (CTLs) to eliminate those T cells. In rheumatoid arthritis (RNA), several specific variable regions of T-cell receptors (TCRs) which are involved in the disease have been characterized. These TCRs include V-3, V-14, V-17 and Vα-17. Thus, delivery of a nucleic acid sequence that encodes at least one of these polypeptides will elicit an immune response that will target T cells involved in RA. In multiple sclerosis (MS), several specific variable regions of TCRs which are involved in the disease have been characterized. These TCRs include V-7 and Vα-10. Thus, delivery of a nucleic acid sequence that encodes at least one of these polypeptides will elicit an immune response that will target T cells involved in MS. In scleroderma, several specific variable regions of TCRs which are involved in the disease have been characterized. These TCRs include V-6, V-8, V-14 and Vα-16, Vα-3C, Vα-7, Vα-14, Vα-15, Va-16, Vα-28 and Vα-12. Thus, delivery of a chimeric adenovirus that encodes at least one of these polypeptides will elicit an immune response that will target T cells involved in scleroderma.

### 3. Delivery Methods

The therapeutic levels, or levels of immunity, of the selected gene can be monitored to determine the need, if any, for boosters. Following an assessment of CD8+T cell response, or optionally, antibody titers, in the serum, optional booster immunizations may be desired. Optionally, the adenoviral vectors of the invention may be delivered in a single administration or in various combination regimens, *e.g.,* in combination with a regimen or course of treatment involving other active ingredients or in a prime-boost regimen. A variety of such regimens have been described in the art and may be readily selected. For example, prime-boost regimens may involve the administration of a DNA (*e*.*g*., plasmid) based vector to prime the immune system to a second or further, booster, administration with a traditional antigen, such as a protein or a recombinant virus carrying the sequences encoding such an antigen. *See*, *e*.*g*., International Patent Publication No. WO 00/11140, published March 2, 2000. Alternatively, an immunization regimen may involve the administration of a modified adenoviral vector of the invention to boost the immune response to a vector (either viral or DNA-based) carrying an antigen, or a protein. In still another alternative, an immunization regimen involves administration of a protein followed by booster with a vector encoding the antigen.

In one embodiment, the invention provides a method of priming and boosting an immune response to a selected antigen in a regimen involving delivery of a modified adenoviral vector of the invention. Such a regimen may involve delivery of a plasmid DNA vector carrying a selected immunogen and/or expression of multiproteins from the prime and/or the boost vehicle. *See, e*.*g*., R.R. Amara, Science, 292:69-74 (6 April 2001) which describes a multiprotein regimen for expression of protein subunits useful for generating an immune response against HIV and SIV. For example, a prime may deliver the Gag, Pol, Vif, VPX and Vpr and Env, Tat, and Rev from a single transcript or multiple transcripts. Alternatively, the SIV Gag, Pol and HIV-1 Env is delivered in a single construct. Still other regimens are described in International Patent Publication Nos. WO 99/16884 and WO 01/54719.

However, the prime-boost regimens are not limited to immunization for HIV or to delivery of these antigens. For example, priming may involve delivering with a first vector followed by boosting with a second vector, or with a composition containing the antigen itself in protein form. In one example, the prime-boost regimen can provide a protective immune response to the virus, bacteria or other organism from which the antigen is derived. In another desired embodiment, the prime-boost regimen provides a therapeutic effect that can be measured using convention assays for detection of the presence of the condition for which therapy is being administered.

The priming composition may be administered at various sites in the body in a dose dependent manner, which depends on the antigen to which the desired immune response is being targeted. The invention is not limited to the amount or situs of injection(s) or to the pharmaceutical carrier. Rather, the regimen may involve a priming and/or boosting step, each of which may include a single dose or dosage that is administered hourly, daily, weekly or monthly, or yearly. As an example, the mammals may receive one or two doses containing between about 10 µg to about 50 µg of plasmid in carrier. A desirable amount of a DNA composition ranges between about 1 µg to about 10,000 µg of the DNA vector. Dosages may vary from about 1 µg to 1000 µg DNA per kg of subject body weight. The amount or site of delivery is desirably selected based upon the identity and condition of the mammal.

The dosage unit of the vector suitable for delivery of the antigen to the mammal is described herein. The vector is prepared for administration by being suspended or dissolved in a pharmaceutically or physiologically acceptable carrier such as isotonic saline; isotonic salts solution or other formulations that will be apparent to those skilled in such administration. The appropriate carrier will be evident to those skilled in the art and will depend in large part upon the route of administration. The compositions of the invention may be administered to a mammal according to the routes described above, in a sustained release formulation using a biodegradable biocompatible polymer, or by on-site delivery using micelles, gels and liposomes. Optionally, the priming step ofthis invention also includes administering with the priming composition, a suitable amount of an adjuvant, such as are defined herein.

Preferably, a boosting composition is administered about 2 to about 27 weeks after administering the priming composition to the mammalian subject. The administration of the boosting composition is accomplished using an effective amount of a boosting composition containing or capable of delivering the same antigen as administered by the priming DNA vaccine. The boosting composition may be composed of a recombinant viral vector derived from the same viral source (*e*.*g*., adenoviral sequences of the invention) or from another source. Alternatively, the "boosting composition" can be a composition containing the same antigen as encoded in the priming DNA vaccine, but in the form of a protein or peptide, which composition induces an immune response in the host. In another embodiment, the boosting composition contains a DNA sequence encoding the antigen under the control of a regulatory sequence directing its expression in a mammalian cell, e.g., vectors such as well-known bacterial or viral vectors. The primary requirements of the boosting composition are that the antigen of the composition is the same antigen, or a cross-reactive antigen, as that encoded by the priming composition.

In another embodiment, the modified adenoviral vectors of the invention are also well suited for use in a variety of other immunization and therapeutic regimens. Such regimens may involve delivery of adenoviral vectors of the invention simultaneously or sequentially with Ad vectors of different serotype capsids, regimens in which adenoviral vectors of the invention are delivered simultaneously or sequentially with non-Ad vectors, regimens in which the adenoviral vectors of the invention are delivered simultaneously or sequentially with proteins, peptides, and/or other biologically useful therapeutic or immunogenic compounds. Such uses will be readily apparent to one of skill in the art.

Thus, the present invention provides immunogenic compositions for use in a variety of treatment and vaccine regimens comprising a modified adenovirus hexon protein of the invention. In one embodiment, a modified adenovirus hexon may be delivered to a subject in the form of an empty modified adenoviral capsid (*i*.*e*., an adenovirus capsid containing the modified adenovirus hexon which does not have packaged therein any minigenes for delivery for the target cell). In another embodiment, a vector expressing this hexon in a target cell may be utilized. In still another embodiment, a modified adenoviral particle carrying a minigene is delivered to a subject.

Such a modified adenoviral particle may contain in its modified capsid a targeting sequence. However, such a modified adenoviral particle may form the basis of a self-priming immunogenic composition. Such a self-priming composition may be prepared when an exogenous amino acid sequence in the adenovirus capsid is an immunogen, and the capsid also has packaged therein a nucleic acid sequence encoding an immunogen. As described herein, the immunogen in the capsid protein and the immunogen contained within the molecule packaged within the capsid may be the same, may induce an immune response to the same virus or organism. In another embodiment, a self-priming adenoviral particle of the invention may contain in its capsid an immunogen which induces non-specific immune response and have packaged therein a said second immunogen which induces a specific or cross-reactive immune response to a selected cell type, molecule or organism.

### Examples

The following examples are illustrative, and are not intended to limit the invention to those illustrated embodiments.

The inventors have demonstrated that a viable adenovirus mutant can be generated that contains a 25 amino acid deletion (ALEINLEEEDDDNEDEVDEQAEQQK, SEQ ID NO: 11) of the hexon (the HVR1 region as defined by Rux, et al, (2003) cited above.

The inventors further demonstrate that an exogenous DNA segment encoding a desired peptide sequence (in this example, the peptide sequence - EQELLELDKWASLW, SEQ ID NO: 12 - corresponding to the section of the HIV envelope protein reported to harbor an HIV neutralization epitope) can be inserted in place of the deletion.

### Example 1: The insertional mutagenesis of the HAdV-5 hexon

The cloning steps undertaken to mutate the DNA sequence of the hexon for it to encode foreign peptide sequences were as follows.

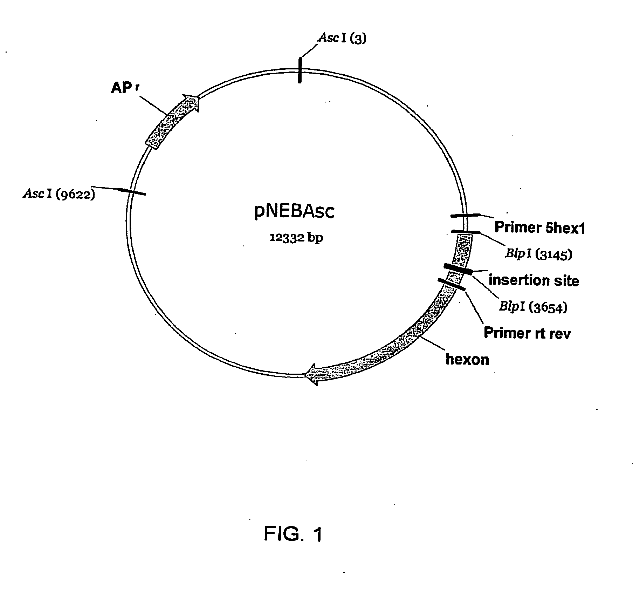

The AscI fragment from the HAdV-5 genome harboring the hexon coding region is subcloned into the plasmid pNEB193 (purchased from New England Biolabs) to create pNEBAsc (see Fig 1).

PCR (polymerase chain reaction) mutagenesis is carried out on the hexon to create a new SpeI site at the insertion site. The details of the PCR mutagenesis to create the SpeI site are as follows.

The PCR template for reactions 1 and 2 are pNEBAsc (the Ad5 AscI fragment containing the hexon region cloned in the plasmid pNEB193). The products of reactions 1 and 2 were combined as used as a template for reaction 3. The PCR enzyme was Tgo polymerase. Cycling for all three reactions was as follows: 94° - 30 sec., 45° - 60 sec., 72°-60 sec., 40 times.

For Reaction 1, primers "5hexl" (GACCGCCGTTGTTGTAACCC, SEQ ID NO: 13) and "It rev" (GTTGTCATCGTCCACTAGTCCCTCTTCTTCTAGGTTTATTTCAAG, SEQ ID NO: 14) were used to amplify a 713 bp fragment.

For Reaction 2, primers "rt fwd" (GGACTAGTGGACGATGACAACGAAGACGA, SEQ ID NO: 15) and "rt rev" (ATGGTTTCATTGGGGTAGTC, SEQ ID NO: 16) were used to amplify a 259 bp fragment.

For Reaction 3, the fragments resulting from Reaction 1 and Reaction 2 were sewn together using the primers "5hexl" and "rt rev" resulting in a 951 bp product. The sewn 951 bp product was cut with BlpI; the resulting 518 bp fragment was used to replace the native BlpI fragment of pNEBAsc to yield pNEBAscSpe. This inserts GGACTAGTG [nt 1 - 9 of SEQ ID NO: 15] (encoding the amino-acids GLV) containing an SpeI site between EEE and DDD (between aa # 149 and 150) in the hexon amino-acid sequence.]

The synthetic DNA fragments encoding the desired peptide sequences were inserted into the SpeI site. The details of the procedure were as follows.

Following annealing the oligomers "CD4 top" (CTAGGCTGCTACGGCGTGAGCGCCACCAAGCTGGGG, SEQ ID NO: 18) and "CD4 bot" (CTAGCCCCAGCTTGGTGGCGCTCACGCCGTAGCAGC, SEQ ID NO: 19) together, a Balb/c mouse CD4 epitope from the spike protein of the SARS coronavirus was inserted into the SpeI site of pNEBAscSpe to yield pNEBAscSpe-spikeCD4. This puts GLGCYGVSATKLGLV (SEQ ID NO: 20) between EEE and DDD (between aa # 149 and 150) of hexon.

To insert a Balb/c mouse CD8 epitope from the spike protein of the SARS coronavirus, the oligomers "CD8 top" (CTAGGGACCAGCACCGGCAACTACAACTACAAGTACCGCTACCTGCGCCACGGC AAGCTGCGCCCCGGG, SEQ ID NO: 21) and "CD8 bot" (CTAGCCCGGGGCGCAGCTTGCCGTGGCGCAGGTAGCGGTACTTGTAGTTGTAGT TGCCGGTGCTGGTCC, SEQ ID NO: 22) were annealed together and inserted into the SpeI site of pNEBAscSpe to yield pNEBAscSpe-spikeCD8. This puts the amino-acid sequence GLGTSTGNYNYKYRYLRHGKLRPGLV (SEQ ID NO: 23) between EEE and DDD (between aa # 149 and 150) of hexon.

The native hexon containing plasmid molecular clone of an E1 and E3 deleted HAdV-5 vector (pSRAd5eGFP) was replaced with the ones containing the hexon with inserted foreign sequences, to create new plasmid molecular clones (pH5sCD4 and pH5sCD8, respectively) harboring the mutated hexon.

PCR (polymerase chain reaction) mutagenesis was carried out on the hexon to create a new BspEI restriction enzyme site at the insertion site. The details of the PCR mutagenesis to create the BspE1 site were as follows.

pNEBAsc (the Ad5 AscI fragment containing the hexon region cloned in the plasmid pNEB 193) was the template for reactions 1 and 2. The products of reactions I and 2 were combined as used as template for reaction 3.

Phusion™ high-fidelity PCR kit purchased from NEB and used according to manufacturer's instructions. Cycling for all three reactions was: 98° - 5 sec., 55° - 15 sec., 72°- 60 sec., 25 times.

For Reaction 1, the primers "5hex1" (GACCGCCGTTGTTGTAACCC, SEQ ID NO: 24) and BspSOErev (CGTGAGTTCCGGAAGTAGCAGCTTCATCCCATTCG, SEQ ID NO: 25) were used to amplify a 678 bp fragment. For Reaction 2, the primers BspSOEfwd (TGCTACTTCCGGAACTCACGTATTTGGGCAGGCG, SEQ ID NO: 26) and 5hex4 (GGAAGAAGGTGGCGTAAAGG, SEQ ID NO: 27) were used to amplify a 1379 bp fragment.

For Reaction 3, the fragments resulting from Reaction 1 and Reaction 2 were sewn together using the primers 5hex1 and 5hex4 and the 2037 bp product cut with RsrII+AvrII; the resulting 1908 bp fragment was ligated into pNEBAsc/ RsrII+AvrII to yield pNEBAseBspEI. This deletes 75 bp encoding ALEINLEEEDDDNEDEVDEQAEQQK [SEQ ID NO: 11] and inserts TCCGGA (nt 71-3 of SEQ ID NO: 27 encoding SG) containing a BspEI site in the hexon sequence.

Synthetic DNA fragments encoding the desired peptide sequences were inserted into the BspeI site. The details of the procedure were as follows. To insert the HIV 2F5 epitope region into pNEBAscBspE1, the oligomers '2F5 top' (CCGGCGAGCAGGAGCTGCTGGAGCTGGACAAGTGGGCCAGCCTGTGGT, SEQ ID NO: 28) and '2F5 bot' (CCGGACCACAGGCTGGCCCACTTGTCCAGCTCCAGCAGCTCCTGCTCG, SEQ ID NO: 17) were annealed and inserted into the BspEI site to yield the plasmid pNEBAsc 2F5. This puts the amino acid sequence ALEINLEEEDDDNBDEVDEQAEQQK (SEQ ID NO: 11) in place of the 25 amino acid deletion carried out above.

The native hexon - containing plasmid molecular clone of an E1 and E3 deleted HAdV-5 vector (pSRAd5eGFP) was replaced with the ones containing the hexon with a deletion or inserted foreign sequences, to create new plasmid molecular clones (pH5hexBspeI and pH5 2F5, respectively) harboring the mutated hexon.

Infectious recombinant HAdV-5 vector was generated with a mutated hexon by transfecting the new plasmid molecular clone harboring the mutated hexon (linearized with PacI) into HEK 293 cells.

This new HAdV-5 vector containing a modified hexon protein may be used in a variety of applications, as described herein.

While the invention has been described with reference to a particularly preferred embodiment, it will be appreciated that modifications can be made within the scope of the appended claims.

## Claims

1. An adenovirus having a capsid comprising a modified adenovirus hexon protein, said modified adenovirus hexon protein comprising a deletion in at least a hypervariable region of an adenovirus hexon protein selected from hypervariable region 1 and hypervariable region 4 and an exogenous amino acid sequence comprising an immunogenic amino acid sequence inserted in the said hypervariable region 1 and/or the said hypervariable region 4, with the proviso that said exogenous amino acid sequence is other than an adenovirus sequence.

2. The adenovirus according to claim 1, wherein said hexon protein comprises more than one deletion and/or a partial deletion.

3. The adenovirus according to claim 1 or 2, wherein the deletion comprises at least 25 amino acids of the native hypervariable region.

4. The adenovirus according to any of claims 1 to 3, wherein at least one amino acid from the N-terminus and at least one amino acid from the C-terminus of the native hypervariable region is retained.

5. The adenovirus according to any of claims 1 to 4, wherein said modified adenovirus hexon protein comprises more than one exogenous amino acid sequence inserted therein.

6. The adenovirus according to any of claims 1 to 5, wherein the exogenous amino acid sequence comprises 5 to 45 amino acid residues in length, or about 25 amino acids in length.

7. The adenovirus according to claim 5 or 6, wherein the exogenous amino acid sequence comprises a targeting sequence.

8. The adenovirus according to claim 7, wherein the targeting sequence is selected from the group consisting of a ligand for a cellular receptor and an epitope for an antibody or a fragment thereof.

9. The adenovirus according to claim 8, wherein the said targeting sequence is selected from the group consisting of a bi-specific antibody, an anti-fiber knob Fab, and an anti-receptor antibody.

10. The adenovirus according to any of claims 1 to 9, wherein said immunogenic amino acid sequence is selected from the group consisting of a T-cell epitope, an antibody epitope, and an antigen from an HIV protein.

11. The adenovirus according to claim 10, wherein said T-cell epitope is a neutralization epitope.

12. The adenovirus according to claim 10, wherein the amino acid sequence is from an HIV protein selected from a tat protein or an envelope protein.

13. The adenovirus according to claim 12, wherein the exogenous amino acid sequence is EQELLELDKWASLW, SEQ ID NO: 12.

14. A method of altering the specificity of an adenovirus vector, said method comprising the step of providing an adenovirus according to claim 1, the modified adenovirus hexon protein of said adenovirus comprising a targeting sequence inserted in the deletion in the hypervariable region.

15. The method according to claim 14, wherein a positively charged amino acid sequence is inserted in the site of the deletion.

16. Use of an adenovirus according to any one of claims 1 to 13 in preparing a medicament.

17. An immunogenic composition comprising a pharmaceutically acceptable carrier and a composition according to any one of claims 1 to 13.

18. A self-priming immunogenic composition comprising:
an adenovirus having a capsid comprising a modified adenovirus hexon protein according to claim 1, wherein said exogenous amino acid sequence is a first immunogen amino acid sequence;
wherein said adenovirus further comprises a nucleic acid sequence encoding a second immunogenic amino acid sequence.

19. The self-priming immunogenic composition according to claim 18, wherein said first immunogenic or antigenic amino acid sequence and said second immunogenic or antigenic amino acid sequence are the same.

20. The self-priming immunogenic composition according to claim 18, wherein said first immunogenic or antigenic amino acid sequence and said second immunogenic or antigenic amino acid sequence differ and induce an immune response to the same virus or organism.

21. The self-priming immunogenic composition according to claim 18, wherein said first immunogenic or antigenic amino acid sequence induces a non-specific immune response and said second immunogenic or antigenic amino acid sequence induces an immune response to a virus or organism.

22. Use of a self-priming immunogenic composition according to any one of claims 18 to 21 in preparing a medicament.

## Patentansprüche

1. Adenovirus mit einem Capsid, das ein modifiziertes Adenovirushexonprotein umfaßt, das seinerseits eine Deletion in wenigstens einer hypervariablen Region eines Adenovirushexonprotein, ausgewählt aus der hypervariablen Region 1 und der hypervariablen Region 4, und eine exogene Azninosäure umfaßt, die ihrerseits eine immunogene Aminosäuresequenz umfaßt, die in die hypervariable Region 1 und/oder die hypervariable Region 4 inseriert ist, mit der Maßgabe, daß sich die exogene Aminosäuresequenz von einer Adenovirussequenz unterscheidet.

2. Adenovirus nach Anspruch 1, wobei das Hexonprotein mehr als eine Deletion und/oder eine teilweise Deletion umfaßt.

3. Adenovirus nach Anspruch 1 order 2, wobei die Deletion wenigstens 25 Aminosäuren der nativen hypervariablen Region umfaßt.

4. Adenovirus nach einem der Ansprüche 1 bis 3, wobei wenigstens eine Aminosäure aus dem N-Terminus und wenigstens eine Aminosäure aus dem C-Terminus der nativen hypervariablen Region erhalten bleibt.

5. Adenovirus nach einem der Ansprüche 1 bis 4, wobei das modifizierte Adenovirushexonprotein mehr als eine exogene, darin inserierte Aminosäuresequenz umfaßt.

6. Adenovirus nach einem der Ansprüche 1 bis 5, wobei die exogene Aminosäuresequenz eine Länge von 5 bis 45 Aminosäureresten bzw. ca. 25 Aminosäuren umfaßt.

7. Adenovirus nach Anspruch 5 oder 6, wobei die exogene Aminosäuresequenz eine Targetingsequenz umfaßt.

8. Adenovirus nach Anspruch 7, wobei die Targetingsequenz ausgewählt ist aus der Gruppe, bestehend aus einem Liganden für einen Zellrezeptor und einem Epitop für einen Antikörper oder ein Fragment davon.

9. Adenovirus nach Anspruch 8, wobei die Targetingsequenz ausgewählt ist aus der Gruppe, bestehend aus einem bispezifischen Antikörper, einem Anti-fiber-knob-Fab und einem Antirezeptorantikörper.

10. Adenovirus nach einem der Ansprüche 1 bis 9, wobei die immunogene Aminosäuresequenz ausgewählt ist aus der Gruppe, bestehend aus einem T-Zellenepitop, einem Antikörperepitop und einem Antigen aus einem HIV-Protein.

11. Adenovirus nach Anspruch 10, wobei das T-Zellenepitop ein Neutralisationsepitop ist.

12. Adenovirus nach Anspruch 10, wobei die Aminosäuresequenz aus einem HIV-Protein stammt, ausgewählt aus einem Tat-Protein oder einem Hüllprotein.

13. Adenovirus nach Anspruch 12, wobei die exogene Aminosäuresequenz EQELLELDKWASLW, SEQ ID NO: 12 ist.

14. Verfahren zur Abänderung der Spezifität eines Adenovimsvektors, das die Stufe der Bereitstellung eines Adenovirus nach Anspruch 1 umfaßt, wobei das modifizierte Adenovirushexonprotein des Adenovirus eine Targetingsequenz umfaßt, die bei der Deletion in die hypervariable Region inseriert wird.

15. Verfahren nach Anspruch 14, bei dem eine positiv geladene Aminosäuresequenz in den Deletionssite inseriert wird.

16. Verwendung eines Adenovirus nach einem der Ansprüche 1 bis 13 zur Herstellung eines Medikaments.

17. Immunogene Zusammensetzung, die einen pharmazeutisch verträglichen Träger und eine Zusammensetzung nach einem der Ansprüche 1 bis 13 umfaßt.

18. Self-priming-immunogene Zusammensetzung, die ein Adenovirus mit einem Capsid, das ein modifiziertes Adenovirushexonprotein nach Anspruch 1 umfaßt, wobei die exogene Aminosäuresequenz eine erste immunogene Aminosäuresequenz ist,
wobei das Adenovirus außerdem noch eine, eine zweite immunogene Aminosäuresequenz kodierende Nukleinsäuresequenz umfaßt.

19. Self-priming-immunogene Zusammensetzung nach Anspruch 18, wobei die erste immunogene oder antigene Aminosäuresequenz und die zweite immunogene oder antigene Aminosäuresequenz identisch sind.

20. Self-priming-immunogene Zusammensetzung nach Anspruch 18, wobei die erste immunogene oder antigene Aminosäuresequenz und die zweite immunogene oder antigene Aminosäuresequenz sich voneinander unterscheiden und auf dasselbe Virus bzw. auf denselben Organismus eine immunantwort auslösen.

21. Self-priming-immunogene Zusammensetzung nach Anspruch 18, wobei die erste immunogene oder antigene Aminosäuresequenz eine nichtspezifische Immunantwort auslöst und die zweite immunogene oder antigene Aminosäuresequenz eine Immunantwort auf ein Virus oder einen Organismus auslöst.

22. Verwendung einer self-priming-immunogenen Zusammensetzung nach einem der Ansprüche 18 bis 21 zur Herstellung eines Medikaments.

## Revendications

1. Adénovirus ayant une capside comprenant une protéine hexon d'adénovirus modifiée, ladite protéine hexon d'adénovirus modifiée comprenant une délétion dans au moins une région hypervariable d'une protéine hexon d'adénovirus choisie parmi la région Hypervariable 1 et la région hypervariable 4 et une séquence d'acides aminés exogène comprenant une séquence d'acides aminés immunogène insérée dans ladite région hypervariable 1 et/ou dans ladite région hypervariable 4, à la condition que ladite séquence d'acides aminés exogène soit différente d'une séquence d'adénovirus.

2. Adénovirus selon la revendication 1, dans lequel ladite protéine hexon comprend plus d'une délétion et/ou d'une délétion partielle.

3. Adénovirus selon la revendication 1 ou 2, dans lequel la délétion comprend au moins 25 acides aminés de la région hypervariable native.

4. Adénovirus selon l'une quelconque des revendications 1 à 3, dans lequel sont conservés au moins un acide aminé provenant du site N-terminal et au moins un acide aminé provenant du site C-terminal de la région hypervariable native.

5. Adénovirus selon l'une quelconque des revendications 1 à 4, dans lequel plus d'une séquence d'acides aminés exogène a été insérée dans ladite protéine hexon d'adénovirus modifiée.

6. Adénovirus selon l'une quelconque des revendications 1 à 5, dans lequel la séquence d'acides aminés exogène a une taille de 5 à 45 résidus d'acides aminés, ou d'environ 25 acides aminés.

7. Adénovirus selon la revendication 5 ou 6, dans lequel la séquence d'acides aminés exogène comprend une séquence cible.

8. Adénovirus selon la revendication 7, dans lequel la séquence cible est choisie dans le groupe consistant en un ligand pour un récepteur cellulaire ou un épitope pour un anticorps ou un fragment de celui-ci.

9. Adénovirus selon la revendication 8, dans lequel ladite séquence cible est choisie dans le groupe consistant en un anticorps bispécifique, un Fab anti-bouton de fibre, et un anticorps anti-récepteur.

10. Adénovirus selon l'une quelconque des revendications 1 à 9, dans lequel ladite séquence d'acides aminés immunogène est choisie dans le groupe consistant en un épitope de cellule T, un épitope d'anticorps et un antigène provenant d'une protéine VIH.

11. Adénovirus selon la revendication 10, dans lequel ledit épitope de cellule T est un épitope de neutralisation.

12. Adénovirus selon la revendication 10, dans lequel la séquence d'acides aminés provent d'une protéine VIH choisie parmi une protéine tat ou une protéine d'enveloppe.

13. Adénovirus selon la revendication 12, dans lequel la séquence d'acides aminés exogène est EQELLELELDKWASLW, SEQ ID N° 12.

14. Procédé de modification de la spécificité d'un vecteur adénovirus, ledit procédé comprenant l'étape consistant à mettre à disposition un adénovirus selon la revendication 1, la protéine hexon d'adénovirus modifiée dudit adénovirus comprenant une séquence cible insérée dans la délétion effectuée dans la région hypervariable.

15. Procédé selon la revendication 14, dans lequel une séquence d'acides aminés positivement chargée est insérée dans le site de la délétion.

16. Utilisation d'un adénovirus selon l'une quelconque des revendications 1 à 13 pour préparer un médicament.

17. Composition immunogène comprenant un support pharmaceutiquement acceptable et une composition selon l'une quelconque des revendications 1 à 13.

18. Composition immunogène à auto-amorçage, comprenant un adénovirus ayant une capside comprenant une protéine hexon d'adénovirus modifiée selon la revendication 1, ladite séquence d'acides aminés exogène étant une première séquence d'acides aminés immunogène, et ledit adénovirus comprenant en outre une séquence d'acides aminés codant pour une deuxième séquence d'acides aminés immunogène.

19. Composition immunogène à auto-amorçage selon la revendication 18, dans laquelle ladite première séquence d'acides aminés immunogène ou antigénique et ladite deuxième séquence d'acides aminés immunogène ou antigénique, sont les mêmes.

20. Composition immunogène à auto-amorçage selon la revendication 18, dans laquelle ladite première séquence d'acides aminés immunogène ou antigénique et ladite deuxième séquence d'acides aminés immunogène ou antigénique sont différentes et induisent une réponse immunitaire au même virus ou au même organisme.

21. Composition immunogène à auto-amorçage selon la revendication 18, dans laquelle ladite première séquence d'acides aminés immunogène ou antigène induit une réponse immunitaire non spécifique, et ladite deuxième séquence d'acides aminés immunogène ou antigène induit une réponse immunitaire à un virus ou à un organisme.

22. Utilisation d'une composition immunogène à auto-amorçage selon l'une quelconque des revendications 18 à 21 pour préparer un médicament.
